# EUROPEAN PATENT SPECIFICATION

(11) **EP 4 397 358 B1**
(45) Date of publication and mention of the grant of the patent: **30.04.2025**
(21) Application number: 22882658.2
(22) Date of filing: 09.10.2022
(51) Int. Cl.: A61N 1/32, A61N 1/06, A61N 1/36, A61N 1/02

(54) **ELECTRIC FIELD GENERATING APPARATUS AND CONTROL METHOD THEREFOR, AND COMPUTER-READABLE STORAGE MEDIUM**
VORRICHTUNG ZUR ERZEUGUNG EINES ELEKTRISCHEN FELDES UND STEUERVERFAHREN DAFÜR SOWIE COMPUTERLESBARES SPEICHERMEDIUM
APPAREIL DE GÉNÉRATION DE CHAMP ÉLECTRIQUE ET PROCÉDÉ DE COMMANDE ASSOCIÉ, ET SUPPORT DE STOCKAGE LISIBLE PAR ORDINATEUR

(30) Priority: 22.10.2021 CN 202111231292
(43) Date of publication of application: 10.07.2024
(73) Proprietor: Hangzhou Wknife Medical Technology Co., Ltd., Hangzhou, Zhejiang 310000 (CN)
(72) Inventor: ZHONG, Xinghua, Hangzhou, Zhejiang 310000 (CN); WANG, Long, Hangzhou, Zhejiang 310000 (CN); YANG, Ke, Hangzhou, Zhejiang 310000 (CN); ZHOU, Libo, Hangzhou, Zhejiang 310000 (CN); TAO, Yinjiong, Hangzhou, Zhejiang 310000 (CN)
(74) Representative: Monteiro Alves, Inês
(86) International application number: PCT/CN2022/124124
(87) International publication number: WO 2023/066048

(56) References cited:
- CN-A- 106 823 145
- CN-A- 109 432 601
- CN-A- 113 082 519
- CN-A- 113 476 746
- CN-A- 113 663 215
- US-A1- 2017 049 513

## Description

### TECHNICAL FIELD

The present application relates to the field of surgery, in particular to an electric field generating device, a control method for the electric field generating device, and a computer-readable storage medium.

### BACKGROUND

Electric field therapy is a therapy implemented through a portable, non-invasive medical device, and its principle is to use a low-intensity, medium-frequency designed electric field to apply to such target biological tissue as microtubules having proliferation and diseased cells, interfere with the mitosis of diseased cells, and cause affected diseased cells to undergo apoptosis and inhibit the growth of the diseased cells.

Currently, in the existing devices used to destroy diseased cells or inhibit the division of diseased cells, an electrical signal generator applies a series of electrical signals to an electrode pair. Since two electrodes in the electrode pair are directly facing each other, a vertical electric field is generated between the two directly facing electrodes of the electrode pair, and the vertical electric field is applied to a target biological tissue region containing target biological tissue.

It has been found through research that a pair of electrodes is located at the periphery of a target biological tissue region, and target biological tissue (such as diseased cells) in the target biological tissue region is not guaranteed to be within the vertical electric field range of the pair of electrodes in the prior art, or is not located entirely within the vertical electric field range of the pair of electrodes in the prior art. That is, the coverage of the vertical electric field in the prior art is too fixed and small, so the coverage of the target biological tissue is limited, and thereby the intensity of the electric field covering a target biological tissue region is limited, and it is ineffective in inhibiting the division of such target biological tissue as diseased cells.
CN 109432601 A discloses a device for destroying and suppressing the rapid growth of diseased tissue in a patient.

### SUMMARY

In view of the shortcomings of the existing methods, the present application provides an electric field generating device, a control method for the electric field generating device, and a computer-readable storage medium, as defined in the appended set of claims, so as to solve the technical problem in the prior art that, it is ineffective in inhibiting the division of such target biological tissue as diseased cells due to the limited intensity of the electric field covering a target biological tissue region, and thereby the intensity of the electric field covering the target biological tissue is limited.

In the technical solution of the present application, n electrodes surround the target biological tissue region in a set manner, and the electrical signal generator is controlled to apply the first electrical signal to m electrodes of the n electrodes, and apply the second electrical signal to at least two electrodes of the n-m electrodes, where n is an integer not less than 3, 1≤m<n, and m is an integer. In the embodiments of the present application, it is able to flexibly select the quantity of electrodes surrounding the target biological tissue region according to a position of the target biological tissue in the target biological tissue region, and control which electrodes to be applied to the second electrical signal, so that a coverage area of the electric field generated between the electrodes with the first electrical signal and the electrodes with the second electrical signal best matches the position of the target biological tissue. Therefore, it is able to improve a matching degree between the coverage area of the electric field and the position of the target biological tissue, a coverage, and flexibility or adaptability of the electric field on the target biological tissue, thereby to increase the intensity of the electric field covering the target biological tissue, and further improve the effect of inhibiting the division of such target biological tissue as diseased cells.

Moreover, multiple electric fields can be generated between the electrodes with the first electrical signal and at least two electrodes with the second electrical signal, and can be superimposed so as to enhance the intensity of an electric field at a superimposed region. When at least two electrodes with the second electrical signal are selected reasonably, it is able to improve the coverage of the electric field on the target biological tissue, thereby to improve the effect of inhibiting the division of such target biological tissue as diseased cells.

The additional aspects and advantages of the present disclosure will be given or may become apparent in the following description, or may be understood through the implementation of the present application.

### BRIEF DESCRIPTION OF THE DRAWINGS

The above and/or additional aspects as well as advantages of the present application will become apparent and are easily understood in the following description with reference to the following drawings. In these drawings,
Fig. 1 is a schematic block diagram of an electric field generating device according to the embodiments of the present application;
Fig. 2 is a schematic block diagram of another electric field generating device according to the embodiments of the present application;
Fig. 3 is a schematic diagram of an electric field generated by yet another electric field generating device where six electrodes are arranged according to the embodiments of the present application;
Fig. 4 is a schematic diagram of a two-dimensional model of a target biological tissue region according to the embodiments of the present application;
Fig. 5a is a schematic diagram of a field strength distribution of a case where a single electrode is grounded in the two-dimensional model of the target biological tissue region according to the embodiments of the present application;
Fig. 5b is a schematic diagram of a field strength distribution of a case where two electrodes are grounded in the two-dimensional model of the target biological tissue region according to the embodiments of the present application;
Fig. 5c is a schematic diagram of a field strength distribution of another case where two electrodes are grounded in the two-dimensional model of the target biological tissue region according to the embodiments of the present application;
Fig. 6 is a schematic diagram of a three-dimensional model of a human thorax according to the embodiments of the present application of the present application;
Fig. 7 is a schematic diagram of a double-layer structure of a single electrode in the three-dimensional model of the human thorax according to the embodiments of the present application of the present application;
Fig. 8a is a schematic view showing the distribution of electrode arrays on the front side, back, and side of the three-dimensional model of the human thorax for human thorax lung cancer treatment according to the embodiments of the present application;
Fig. 8b is a schematic view showing the distribution of the electrode arrays on the back of the three-dimensional model of the human thorax for the human thorax lung cancer treatment according to the embodiments of the present application;
Figs. 9a to 9d are schematic views showing sizes and location distributions of four virtual tumor models in the three-dimensional model of the human thorax according to the embodiments of the present application;
Fig. 10 is curve diagram between the electric field intensity and volume of the three-dimensional model of the human thorax according to the embodiments of the present application;
Fig. 11a is a schematic view showing the distribution of the electric field in a horizontal plane of the thorax in a case where a single electrode array on the back of the three-dimensional model of the human thorax including Tumor T1 is grounded according to the embodiments of the present application;
Fig. 11b is a schematic view showing the distribution of the electric field in the horizontal plane of the thorax in a case where two electrode arrays respectively on the left side and back of the three-dimensional model of the human thorax including the tumor T1 are grounded according to the embodiments of the present application;
Fig. 11c is a schematic view showing the distribution of the electric field in the horizontal plane of the thorax in a case where two electrode arrays on the back of the three-dimensional model of the human thorax including Tumor T1 are grounded according to the embodiments of the present application;
Fig. 12a is a schematic view showing the distribution of the electric field in a horizontal plane of the thorax in a case where a single electrode array on the back of the three-dimensional model of the human thorax including Tumor T2 is grounded according to the embodiments of the present application;
Fig. 12b is a schematic view showing the distribution of the electric field in the horizontal plane of the thorax in a case where two electrode arrays respectively on the left side and back of the three-dimensional model of the human thorax including Tumor T2 are grounded according to the embodiments of the present application;
Fig. 12c is a schematic view showing the distribution of the electric field in the horizontal plane of the thorax in a case where two electrode arrays on the back of the three-dimensional model of the human thorax including Tumor T2 are grounded according to the embodiments of the present application;
Fig. 13a is a schematic view showing the distribution of the electric field in a horizontal plane of the thorax in a case where a single electrode array on the back of the three-dimensional model of the human thorax including Tumor T3 is grounded according to the embodiments of the present application;
Fig. 13b is a schematic view showing the distribution of the electric field in the horizontal plane of the thorax in a case where two electrode arrays respectively on the right side and back of the three-dimensional model of the human thorax including Tumor T3 are grounded according to the embodiments of the present application;
Fig. 13c is a schematic view showing the distribution of the electric field in the horizontal plane of the thorax in a case where two electrode arrays on the back of the three-dimensional model of the human thorax including Tumor T3 are grounded according to the embodiments of the present application;
Fig. 14a is a schematic view showing the distribution of the electric field in a horizontal plane of the thorax in a case where a single electrode array on the back of the three-dimensional model of the human thorax including Tumor T4 is grounded according to the embodiments of the present application;
Fig. 14b is a schematic view showing the distribution of the electric field in the horizontal plane of the thorax in a case where two electrode arrays respectively on the right side and back of the three-dimensional model of the human thorax including Tumor T4 are grounded according to the embodiments of the present application; and
Fig. 14c is a schematic view showing the distribution of the electric field in the horizontal plane of the thorax in a case where two electrode arrays on the back of the three-dimensional model of the human thorax including Tumor T4 are grounded according to the embodiments of the present application.

### Reference Sign List

10-n electrodes, 25-electrical signal generator, 20-first electric signal generating circuit, 30-second electric signal generating circuit, 40-control signal generator, 50-first switch assembly, 501-first switch unit, 60-second switch assembly, 601-second switch unit, 70-multi-channel analog switch unit;
80-target biological tissue region, 81-target biological tissue, 82-normal biological tissue; 91-first layer, 92-second layer, 201-first electrode array, 202-second electrode array, 203-third electrode array.

### DETAILED DESCRIPTION

The present application provides an electric field generating device, a control method for the electric field generating device and a computer-readable storage medium, so as to solve the technical problem in the prior art that, it is ineffective in inhibiting the division of such target biological tissue as diseased cells due to the limited intensity of the electric field covering a target biological tissue region, and thereby the intensity of the electric field covering the target biological tissue is limited.

The present application will be described hereinafter in conjunction with the embodiments and the drawings. Identical or similar reference numbers in the drawings represent an identical or similar element or elements having an identical or similar function. In addition, the detailed description about any known technology will be omitted when it is unnecessary to the features in the present application. The following embodiments are for illustrative purposes only, but shall not be used to limit the scope of the present application as defined by the appended claims.

As can be appreciated by a person skilled in the art, unless otherwise defined, all terms (including technical and scientific terms) used herein have the same meaning as commonly understood by one of ordinary skill in the art to which the present application is a part. Terms, such as those defined in commonly used dictionaries, should be interpreted as having a meaning that is consistent with their meaning in the context of the relevant art and will not be interpreted in an idealized or overly formal sense, unless expressly so defined herein.

As used herein, the singular forms, "a," "an," and "the" are intended to include the plural forms as well, unless the context clearly indicates otherwise. Moreover, the terms "comprises," "comprising," "includes," and/or "including," when used in this specification, specify the presence of stated features, integers, steps, operations, elements, components, and/or groups thereof, but do not preclude the presence or addition of one or more other features, integers, steps, operations, elements, components, and/or groups thereof. In the case that one element is connected or coupled to another element, it may be directly connected or coupled to the other element, or an intermediate element may be arranged therebetween. At this time, the element may be connected or coupled to the other element in a wireless or wired manner. In addition, the expression "and/or" is used to indicate the existence of all or any one of one or more of listed items, or combinations thereof.

It has been found through research that a pair of electrodes is located at the periphery of a target biological tissue region, and target biological tissue (such as diseased cells) in the target biological tissue region is not guaranteed to be within the vertical electric field range of the pair of electrodes in the prior art, or is not located entirely within the vertical electric field range of the pair of electrodes in the prior art. That is, the coverage of the vertical electric field in the prior art is too fixed and small, so the coverage of the target biological tissue is limited, and thereby the intensity of the electric field covering a target biological tissue region is limited, and it is ineffective in inhibiting the division of such target biological tissue as diseased cells.

The present application provides a target electric field generating device and a control method for the electric field generating device, so as to solve the above technical problems in the prior art.

The technical solution of the present application and how to solve the above technical problems by using the technical solution of the present application will be described in detail hereinafter in conjunction with specific embodiments. The following specific embodiments can be combined with each other, and same or similar concepts or processes may not be reiterated in some embodiments. The embodiments of the present application will be described hereinafter with reference to the accompanying drawings.

The electric field generating device in the embodiments of the present application is for surgical use and is applicable for surgical medical instruments.

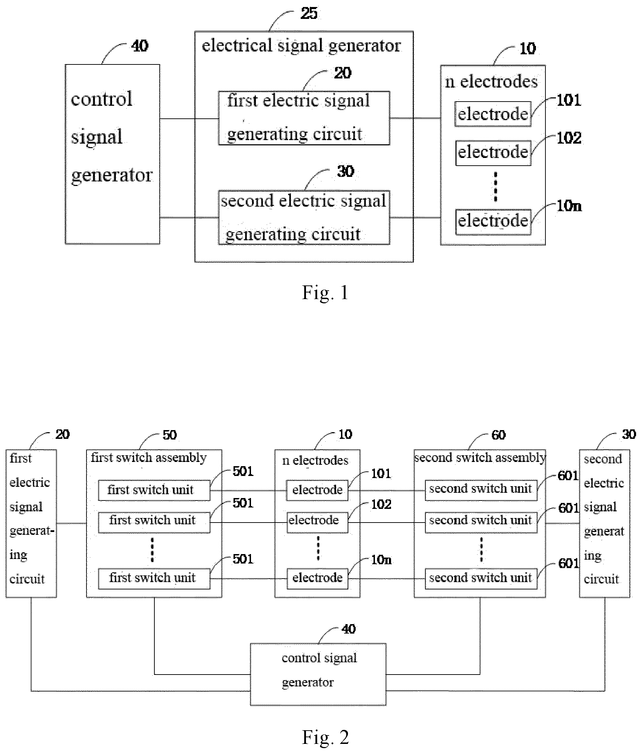

The present application provides a target electric field generating device, as shown in Fig. 1, the electric field generating device includes n electrodes 10, an electrical signal generator 25 and a control signal generator 40. In specific, n electrodes 10 are configured to surround a target biological tissue region in a set manner; where n is an integer not less than 3. The target biological tissue region includes the target biological tissue and a normal biological tissue, and the target biological tissue includes diseased cells, a tumor or a lesion, etc. The lesion refers to a diseased part of a body, or a localized and diseased tissue in the body that contains pathogenic microorganisms. For example, if a certain part of a lung is destroyed by tuberculosis bacteria, this part is a tuberculosis lesion. The normal biological tissue refers to a biological tissue that does not contain diseased cells, a tumor and a lesion, and can be considered as a biological tissue other than the target biological tissue.

The electrical signal generator 25 is electrically connected to the n electrodes 10.

The control signal generator 40 is electrically connected to the electrical signal generator 25, and configured to control the electrical signal generator 25 to apply a first electrical signal to m electrodes of the n electrodes 10, and apply a second electrical signal to at least two electrodes of n-m electrodes, to generate an electric field between the electrodes with the first electrical signal and the electrodes with the second electrical signal, where a voltage of the second electrical signal is less than a voltage of the first electrical signal, 1≤m<n, and m is an integer.

Optionally, the first electrical signal and the second electrical signal may be outputted by one electrical signal generator, or may be outputted by two electrical signal generators respectively.

Optionally, the n electrodes 10 may be attached to a target part of a human body or an animal body according to the set manner.

Optionally, there is at least one electrode with the first electrical signal and at least two electrodes with the second electrical signal. According to the claimed invention, the quantity of electrodes with the first electrical signal is smaller than the quantity of electrodes with the second electrical signal.

Optionally, the control signal generator 40 may be a central processing unit (Central Processing Unit, CPU), a general-purpose processor, a data signal processor (Digital Signal Processor, DSP), an application specific integrated circuit (Application Specific Integrated Circuit, ASIC), or a field-programmable gate array (Field-Programmable Gate Array, FPGA) or other programmable logic devices, transistor logic devices, hardware members or any combination thereof. It may implement or execute various illustrative logical blocks, modules and circuits described in connection with the content of the present application. The control signal generator 40 may also be a combination that implements computing functions, such as a combination of one or more microprocessors, a combination of a DSP and a microprocessor, etc.

In the embodiments of the present application, it is able to flexibly select the quantity of electrodes surrounding the target biological tissue region according to a position of the target biological tissue in the target biological tissue region, and control which electrodes to be applied to the second electrical signal, so that a coverage area of the electric field generated between the electrode with the first electrical signal and the electrodes with the second electrical signal best matches the position of the target biological tissue. Therefore, it is able to improve a matching degree between the coverage area of the electric field and the position of the target biological tissue, a coverage, and flexibility or adaptability of the electric field on the target biological tissue, thereby to increase the intensity of the electric field covering the target biological tissue, and further improve the effect of inhibiting the division of such target biological tissue as diseased cells.

Moreover, multiple electric fields can be generated between the electrode with the first electrical signal and at least two electrodes with the second electrical signal, and can be superimposed so as to enhance the intensity of an electric field at a superimposed region. When at least two electrodes with the second electrical signal are selected reasonably, it is able to improve the coverage of the electric field on the target biological tissue, thereby to improve the effect of inhibiting the division of such target biological tissue as diseased cells.

In some embodiments, as shown in Fig. 1, the electrical signal generator 25 includes a first electrical signal generating circuit 20 and a second electrical signal generating circuit 30. The first electrical signal generating circuit 20 is electrically connected to the n electrodes, and configured to output the first electrical signal. The second electrical signal generating circuit 30 is electrically connected to the n electrodes and configured to output the second electrical signal.

In some embodiments, as shown in Fig. 2, the electric field generating device further includes a first switch assembly 50 and a second switch assembly 60. The first electrical signal generating circuit 20 is electrically connected to the n electrodes 10 through the first switch assembly 50, and the control signal generator 40 is electrically connected to the first switch assembly 50. The second electrical signal generating circuit 30 is electrically connected to the n electrodes 10 through the second switch assembly 60, and the control signal generator 40 is electrically connected to the second switch assembly 60. The control signal generator 40 is configured to control the first switch assembly 50 to transmit the first electrical signal to m electrodes of the n electrodes 10, and control the second switch assembly 60 to transmit the second electrical signal to at least two electrodes of n-m electrodes, to generate the electric field between the electrodes with the first electrical signal and the electrodes with the second electrical signal.

In some embodiments, as shown in Fig. 2, the first switch assembly 50 includes n first switch units 501, and the second switch assembly 60 includes n second switch units 601. The n first switch units 501 are electrically connected to the n electrodes 10 respectively, and electrically connected to the first electrical signal generating circuit 20 and the control signal generator 40. The n second switch units 601 are electrically connected to the n electrodes 10 respectively, and electrically connected to the second electrical signal generating circuit 30 and the control signal generator 40.

Optionally, the n first switch units include a first one of the first switch units to an nth one of the first switch units, the n second switch units include a first one of the second switch units to an nth one of the second switch units, and the n electrodes include a first electrode to an nth electrode.

The first one to the nth one of the first switch units and the first one to the nth one of the second switch units are electrically connected to n electrodes respectively, i.e., the first one of the first switch units and the first one of the second switch units are electrically connected to the first electrode, a second one of the first switch units and a second one of the second switch units are electrically connected to the second electrode, ..., the nth one of the first switch units and the nth one of the second switch units are electrically connected to the nth electrode.

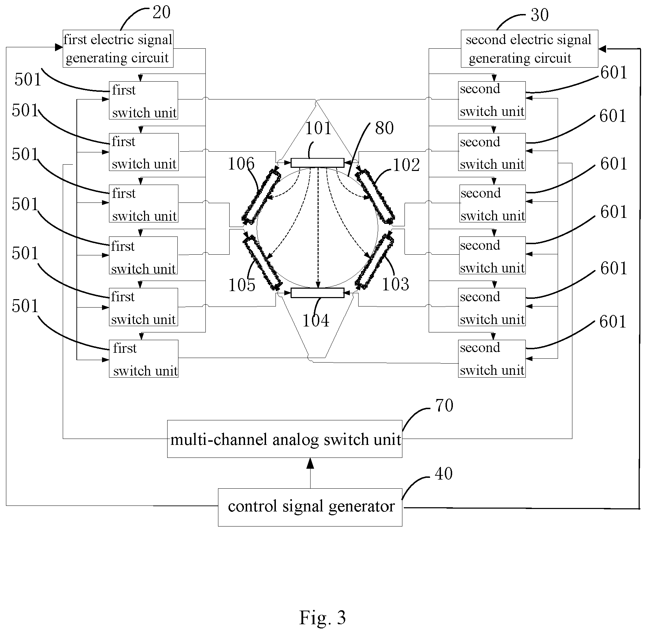

Optionally, as shown in Fig. 3, the electric field generating device further includes a multi-channel analog switch unit 70 electrically connected to n first switch units 501, n second switch units 601, and the control signal generator 40 and configured to control switching of the transmission path of the control signal outputted by the control signal generator 40. The multi-channel analog switch unit 70 has such advantages as fast switching speed, no jitter, low power consumption, small size, reliable operation and being easy to be controlled.

In some embodiments, the electric field generating device further includes at least one of the following:
the first electrical signal including an Alternating Current (AC) voltage signal, a pulse voltage signal or a square wave voltage signal;
the second electrical signal including a constant voltage signal or a fluctuating voltage signal;
an absolute value of a voltage amplitude of the first electrical signal being not less than 0 V and not greater than 500 V;
an absolute value of a voltage amplitude of the second electrical signal being not less than 0 V and not greater than 10 V;
an intensity of the electric field being not less than 0.1 V/cm and not greater than 10 V/cm;
a frequency of the electric field being not less than 50 kilohertz and not greater than 500 kilohertz;
m being 1.

The constant voltage signal means that a size of the voltage is maintained, the fluctuating voltage signal means that the sum of absolute values of positive and negative deviations of the voltage does not exceed 10% of a rated value.

Optionally, the first electrical signal generating circuit 20 includes an alternating current signal generating circuit, a pulse electrical signal generating circuit or a square wave electrical signal generating circuit, the alternating current signal generating circuit is used to output an AC voltage signal, the pulse electrical signal generating circuit is used to output a pulse voltage signal, and the square wave electrical signal generating circuit is used to output a square wave voltage signal.

In some embodiments, the absolute value of the voltage amplitude of the second electrical signal is 0V, 1V or 5V.

As shown in Fig. 3, n=6 is set for illustrative purposes only. As can be appreciated, n may also be any other quantities, such as 20, 30, 50, which is not particularly defined in the present application. The electric field generating device is used to apply an electric field to a target biological tissue region 80, the target biological tissue region 80 may be a target region of a patient. In Fig. 3, there are six electrodes (electrode 101, electrode 102, electrode 103, electrode 104, electrode 105 and electrode 106) surrounding the target biological tissue region 80 (e.g., the electrodes 101-106 may be arranged to be in contact with the patient's body).

Specifically, the first electrical signal generating circuit 20 is electrically connected to six electrodes and used to apply the first electrical signal to the six electrodes. The second electrical signal generating circuit 30 is electrically connected to the six electrodes and used to apply the second electrical signal to the six electrodes.

Optionally, the control signal generator 40 controls one of the first switch units 501 to transmit the first electrical signal to the electrode 101, and controls the remaining first switch units 501 to be turned off. At the same time, the control signal generator 40 controls one of second switch units 601 that is electrically connected to the electrode 101 to be turned off, and controls the remaining second switch units 601 to transmit the second electrical signal to the electrodes 102-106.

For example, the electrode 101 is controlled to have the first electrical signal, and the electrodes 102-106 are controlled to have the second electrical signal, so a first electric field is generated between the electrode 101 and the electrode 102, a second electric field is generated between the electrode 101 and the electrode 103, a third electric field is generated between the electrode 101 and the electrode 104, a fourth electric field is generated between the electrode 101 and the electrode 105, and a fifth electric field is generated between the electrode 101 and the electrode 106. The first electrical signal applied to the electrode 101 is controlled to be a high voltage signal (for example, between 0 and 500V), and the second electrical signal applied to the electrodes 102-106 is controlled to be a low voltage signal (for example, 0 to 10V), that is, there are multiple potential differences between the electrode 101 and the electrodes 102-106, and multiple electric fields are generated between the electrodes 101 and the electrodes 102-106, so it is able to increase the coverage area of the electric field on the patient's target region, and thereby increase the intensity of the electric field covering the patient's target region, increase the intensity of the electric field covering the target biological tissue, where the target biological tissue region includes the target biological tissue and the normal biological tissue, and improve the effect of inhibiting the division of such target biological tissue as diseased cells.

As can be appreciated, it is able to control the electrode 101 and the electrode 102 to have the first electrical signal, and the electrodes 103-106 to have the second electrical signal, or control the electrode 101 to have the first electrical signal, and the electrode 103 and the electrode 105 to have the second electrical signal, or control the electrode 101 to have the first electrical signal, and the electrode 102, the electrode 104 and the electrode 106 to have the second electrical signal, or control the electrode 101 to have the first electrical signal, and the electrode 103, the electrode 104 and the electrode 105 to have the second electrical signal, which is not particularly defined in the present application.

In the embodiments of the present application, it is able to flexibly select the quantity of electrodes surrounding the target biological tissue region according to a position of the target biological tissue in the target biological tissue region, and control which electrodes to be applied to the second electrical signal, so that a coverage area of the electric field generated between the electrode with the first electrical signal and the electrodes with the second electrical signal best matches the position of the target biological tissue. Therefore, it is able to improve a matching degree between the coverage area of the electric field and the position of the target biological tissue, a coverage, and flexibility or adaptability of the electric field on the target biological tissue, thereby to increase the intensity of the electric field covering the target biological tissue, and further improve the effect of inhibiting the division of such target biological tissue as diseased cells.

Moreover, multiple electric fields can be generated between the electrode with the first electrical signal and at least two electrodes with the second electrical signal, and can be superimposed so as to enhance the intensity of an electric field at a superimposed region. When at least two electrodes with the second electrical signal are selected reasonably, it is able to improve the coverage of the electric field on the target biological tissue, thereby to improve the effect of inhibiting the division of such target biological tissue as diseased cells.

Optionally, still referring to Fig. 3, one of the first switch units 501 is controlled to transmit the first electrical signal to the electrode 101, and the remaining first switch units 501 are controlled to be turned off. At the same time, one of the second switch units 601 that is electrically connected to the electrode 101 is controlled to be turned off, and the remaining second switch units 601 are synchronously controlled to be turned on sequentially according to a set order, so as to transmit the second electrical signal sequentially to the electrodes 102-106 according to the set order. That is, the electrode 101 is applied with the first electrical signal continuously, and the electrodes 102-106 are applied with the second electrical signal sequentially according to the set order (that is, the second electrical signal is switched among the electrodes 102-106 according to the set order).

Optionally, the set order includes: a clockwise order, a counterclockwise order, an n-pointed star shape order, or any other hop or non-continuous orders.

Optionally, a time interval for the second switch units 601 to be turned on or off sequentially according to the set order is not less than 20ms and not greater than 500ms.

Optionally, the electrode 101 is controlled to be applied with the first electrical signal within a first time period, the electrode 102 is controlled to be applied with the second electrical signal within a second time period, the electrode 103 is controlled to be applied with the second electrical signal within a third time period, the electrode 104 is controlled to be applied with the second electrical signal within a fourth time period, the electrode 105 is controlled to be applied with the second electrical signal within the fifth time period, and the electrode 106 is controlled to be applied with the second electrical signal within the sixth time period. The first time period, the second time period, the third time period, the fourth time period, the fifth time period and the sixth time period may be all the same, all different, or partially the same, and may be set according to actual situations, which is not particularly defined in the present application.

In the above-mentioned embodiment, the electrode 101 is controlled to be applied with the first electrical signal continuously, and the remaining electrodes 102-106 are controlled to be applied with the second electrical signal sequentially according to the set order, where the voltage of the second electrical signal applied to the remaining electrodes 102-106 is smaller than the voltage of the first electrical signal. As compared with applying the first electrical signal to all electrodes and switching the first electrical signal among all electrodes, in the embodiment of the present application, one electrode is applied with the first electrical signal, the remaining electrodes are applied with the second electrical signal, and the second electrical signal is switched among the remaining electrodes, so the power consumption is reduced, thereby reducing the power consumption of the electric field generating device and extending the standby duration of the battery used for power supply of the electric field generating device.

The electric field generated by the electric field generating device is used to destroy diseased cells or inhibit the division of diseased cells, and the electric field is referred as a tumor treating field (Tumor Treating Field, TTF) in the present application. TTF can inhibit the proliferation of rapidly dividing active cells, such as cancer cells, and disrupt these active cells.

Optionally, still referring to Fig. 3, in order to prevent the electric field generating device from overheating, six first switch units 501 are controlled to be turned on sequentially according to the set order, so as to transmit the first electrical signal to the corresponding electrodes sequentially according to the set order. Six second switch units 601 electrically connected to the corresponding electrodes are synchronously controlled to be turned off sequentially according to the set order, and second switch units 601 that are not turned off are synchronously controlled to be turned on sequentially according to the set order, so as to transmit the second electrical signal to electrodes that do not receive the first electrical signal sequentially according to the set order.

Optionally, the set order includes: a clockwise order, a counterclockwise order, an n-pointed star shape order, or any other hop or non-continuous orders.

Optionally, a time interval for the second switch units 601 to be turned on or off sequentially according to the set order is not less than 20ms and not greater than 500ms.

Optionally, the time at which each electrode has the first electrical signal or the second electrical signal may be set according to actual situations, which is not particularly defined in the present application.

Thus, the electrodes 101-106 are applied with the first electrical signal and the second electrical signal sequentially according to the set order, which further reduces the power consumption of the electric field generating device, thereby preventing the electric field generating device from overheating.

Based on the same inventive concept, the present application provides in some embodiments a control method for the above-mentioned electric field generating device, including:
controlling the electrical signal generator to apply a first electrical signal to m electrodes of the n electrodes, and apply a second electrical signal to at least two electrodes of n-m electrodes, to generate an electric field between the electrodes with the first electrical signal and the electrodes with the second electrical signal; where n is an integer not less than 3, 1≤m<n, m is an integer;
where the electric field generating device includes the electrical signal generator and the n electrodes that are electrically connected to each other; a voltage of the second electrical signal is less than a voltage of the first electrical signal.

In the control method for the electric field generating device of the embodiments of the present application, n electrodes surround the target biological tissue region in a set manner, and the electrical signal generator is controlled to apply the first electrical signal to m electrodes of the n electrodes, and apply the second electrical signal to at least two electrodes of the n-m electrodes, where n is an integer not less than 3, 1≤m<n, and m is an integer. In the embodiments of the present application, it is able to flexibly select the quantity of electrodes surrounding the target biological tissue region according to a position of the target biological tissue in the target biological tissue region, and control which electrodes to be applied to the second electrical signal, so that a coverage area of the electric field generated between the electrode with the first electrical signal and the electrodes with the second electrical signal best matches the position of the target biological tissue. Therefore, it is able to improve a matching degree between the coverage area of the electric field and the position of the target biological tissue, a coverage, and flexibility or adaptability of the electric field on the target biological tissue, thereby to increase the intensity of the electric field covering the target biological tissue, and further improve the effect of inhibiting the division of such target biological tissue as diseased cells.

In addition, multiple electric fields can be generated between the electrode with the first electrical signal and at least two electrodes with the second electrical signal, and can be superimposed so as to enhance the intensity of an electric field at a superimposed region. When at least two electrodes with the second electrical signal are selected reasonably, it is able to improve the coverage of the electric field on the target biological tissue, thereby to improve the effect of inhibiting the division of such target biological tissue as diseased cells.

In some embodiments, controlling the electrical signal generator to apply the first electrical signal to m electrodes of the n electrodes, and apply the second electrical signal to at least two electrodes of the n-m electrodes, to generate the electric field between the electrodes with the first electrical signal and the electrodes with the second electrical signal includes:
controlling a first switch assembly to transmit the first electrical signal to m electrodes of the n electrodes, and controlling a second switch assembly to transmit the second electrical signal to at least two electrodes of the n-m electrodes, to generate the electric field between the electrodes with the first electrical signal and the electrodes with the second electrical signal;
where the electrical signal generator includes a first electrical signal generating circuit and a second electrical signal generating circuit, the electric field generating device further includes the first switch assembly and the second switch assembly, the first switch assembly is electrically connected to the first electrical signal generating circuit, the control signal generator and n electrodes, and the second switch assembly is electrically connected to the second electrical signal generating circuit, the control signal generator and n electrodes.

In some embodiments, controlling the first switch assembly to transmit the first electrical signal to m electrodes of the n electrodes, and controlling the second switch assembly to transmit the second electrical signal to at least two electrodes of the n-m electrodes, to generate the electric field between the electrodes with the first electrical signal and the electrodes with the second electrical signal includes:
controlling a first one of first switch units to transmit the first electrical signal to a first electrode of n electrodes, and controlling a second one of the first switch units to a nth one of the first switch units to be turned off; simultaneously controlling a first one of second switch units that is electrically connected to the first electrode to be turned off, and controlling at least two second switch units among a second one of the second switch units to an nth one of the second switch units to transmit the second electrical signal to at least two electrodes of n electrodes except the first electrode.

The first switch assembly includes n first switch units, and the second switch assembly includes n second switch units. The n first switch units include the first one of the first switch units to the nth one of the first switch units, the n second switch units include the first one of the second switch units to the nth one of the second switch units, and the n electrodes include the first electrode to an nth electrode.

The first one to the nth one of the first switch units and the first one to the nth one of the second switch units are electrically connected to n electrodes respectively, i.e., the first one of the first switch units and the first one of the second switch units are electrically connected to the first electrode, a second one of the first switch units and a second one of the second switch units are electrically connected to the second electrode, ..., the nth one of the first switch units and the nth one of the second switch units are electrically connected to the nth electrode.

As shown in Fig. 3, n=6 is set for illustrative purposes only. As can be appreciated, n may also be any other quantities, such as 20, 30, 50, which is not particularly defined in the present application. The electric field generating device is used to apply an electric field to a target biological tissue region 80, the target biological tissue region 80 may be a target region of a patient. In Fig. 3, there are six electrodes (electrode 101, electrode 102, electrode 103, electrode 104, electrode 105 and electrode 106) surrounding the target biological tissue region 80 (e.g., the electrodes 101-106 may be arranged to be in contact with the patient's body). In Fig. 3, there are six first switch units 501, namely, a first one to a sixth one of the first switch units. There are six second switch units 601 in Fig. 3, namely, a first one to a sixth one of the second switch units.

Specifically, the first electrical signal generating circuit 20 is electrically connected to six electrodes and used to apply the first electrical signal to the six electrodes. The second electrical signal generating circuit 30 is electrically connected to the six electrodes and used to apply the second electrical signal to the six electrodes.

Optionally, the control signal generator 40 controls one of the first switch units 501 to transmit the first electrical signal to the electrode 101, and controls the remaining first switch units 501 to be turned off. At the same time, the control signal generator 40 controls one of second switch units 601 that is electrically connected to the electrode 101 to be turned off, and controls the remaining second switch units 601 to transmit the second electrical signal to the electrodes 102-106.

For example, the electrode 101 is controlled to have the first electrical signal, and the control electrodes 102-106 are controlled to have the second electrical signal, so a first electric field is generated between the electrode 101 and the electrode 102, a second electric field is generated between the electrode 101 and the electrode 103, a third electric field is generated between the electrode 101 and the electrode 104, a fourth electric field is generated between the electrode 101 and the electrode 105, and a fifth electric field is generated between the electrode 101 and the electrode 106.

The first electrical signal applied to the electrode 101 is controlled to be a high voltage signal (for example, between 0 and 500V), and the second electrical signal applied to the electrodes 102-106 is controlled to be a low voltage signal (for example, 0 to 10V), that is, there are multiple potential differences between the electrode 101 and the electrodes 102-106, multiple electric fields are generated between the electrodes 101 and the electrodes 102-106, and multiple electric fields can be superimposed so as to enhance the intensity of an electric field at a superimposed region. When at least two electrodes with the second electrical signal are selected reasonably, it is able to improve the coverage of the electric field on the target biological tissue, thereby to improve the effect of inhibiting the division of such target biological tissue as diseased cells.

As can be appreciated, it is also able to control the electrode 101 and the electrode 102 to have the first electrical signal, and the electrodes 103-106 to have the second electrical signal, or control the electrode 101 to have the first electrical signal, and the electrode 103 and the electrode 105 to have the second electrical signal, or control the electrode 101 to have the first electrical signal, and the electrode 102, the electrode 104 and the electrode 106 to have the second electrical signal, or control the electrode 101 to have the first electrical signal, and the electrode 103, the electrode 104 and the electrode 105 to have the second electrical signal, which is not particularly defined in the present application.

In the embodiments of the present application, it is able to flexibly select the quantity of electrodes surrounding the target biological tissue region according to a position of the target biological tissue in the target biological tissue region, and control which electrodes to be applied to the second electrical signal, so that a coverage area of the electric field generated between the electrode with the first electrical signal and the electrodes with the second electrical signal best matches the position of the target biological tissue. Therefore, it is able to improve a matching degree between the coverage area of the electric field and the position of the target biological tissue, a coverage, and flexibility or adaptability of the electric field on the target biological tissue, thereby to increase the intensity of the electric field covering the target biological tissue, and further improve the effect of inhibiting the division of such target biological tissue as diseased cells.

In some embodiments, controlling the first switch assembly to transmit the first electrical signal to m electrodes of the n electrodes, and controlling the second switch assembly to transmit the second electrical signal to at least two electrodes of the n-m electrodes, to generate the electric field between the electrodes with the first electrical signal and the electrodes with the second electrical signal includes:
controlling a first one of first switch units to transmit the first electrical signal to a first electrode of n electrodes, and controlling a second one of the first switch units to an nth one of the first switch units to be turned off; simultaneously controlling a first one of second switch units that is electrically connected to the first electrode to be turned off, and synchronously controlling at least two second switch units among a second one of the second switch units to an nth one of the second switch units to be turned on sequentially according to a set order, to transmit the second electrical signal to at least two electrodes of n electrodes except the first electrode according to the set order.

The first switch assembly includes n first switch units, and the second switch assembly includes n second switch units. The n first switch units include the first one of the first switch units to the nth one of the first switch units, the n second switch units include the first one of the second switch units to the nth one of the second switch units, and the n electrodes include the first electrode to an nth electrode.

The first one to the nth one of the first switch units and the first one to the nth one of the second switch units are electrically connected to n electrodes respectively, i.e., the first one of the first switch units and the first one of the second switch units are electrically connected to the first electrode, a second one of the first switch units and a second one of the second switch units are electrically connected to the second electrode, ..., the nth one of the first switch units and the nth one of the second switch units are electrically connected to the nth electrode.

In the above-mentioned embodiment, one of n electrodes is controlled to be applied with the first electrical signal continuously, and the remaining electrodes 102-106 are controlled to be applied with the second electrical signal sequentially according to the set order, where the voltage of the second electrical signal applied to the remaining electrodes 102-106 is smaller than the voltage of the first electrical signal. As compared with applying the first electrical signal to all electrodes and switching the first electrical signal among all electrodes, in the embodiment of the present application, one electrode is applied with the first electrical signal, the remaining electrodes are applied with the second electrical signal, and the second electrical signal is switched among the remaining electrodes, so the power consumption is reduced, thereby reducing the power consumption of the electric field generating device.

By way of example, still referring to Fig. 3, one of the first switch units 501 is controlled to transmit the first electrical signal to the electrode 101, and the remaining first switch units 501 are controlled to be turned off. At the same time, one of the second switch units 601 that is electrically connected to the electrode 101 is controlled to be turned off, and the remaining second switch units 601 are synchronously controlled to be turned on sequentially according to the set order, so as to transmit the second electrical signal sequentially to the electrodes 102-106 according to the set order. That is, the electrode 101 is applied with the first electrical signal continuously, and the electrodes 102-106 are applied with the second electrical signal sequentially according to the set order (that is, the second electrical signal is switched among the electrodes 102-106 according to the set order).

Optionally, the set order includes: a clockwise order, a counterclockwise order, an n-pointed star shape order, or any other hop or non-continuous orders.

Optionally, a time interval for the second switch units 601 to be turned on or off sequentially according to the set order is not less than 20ms and not greater than 500ms.

Optionally, the electrode 101 is controlled to be applied with the first electrical signal within a first time period, the electrode 102 is controlled to be applied with the second electrical signal within a second time period, the electrode 103 is controlled to be applied with the second electrical signal within a third time period, the electrode 104 is controlled to be applied with the second electrical signal within a fourth time period, the electrode 105 is controlled to be applied with the second electrical signal within the fifth time period, and the electrode 106 is controlled to be applied with the second electrical signal within the sixth time period. The first time period, the second time period, the third time period, the fourth time period, the fifth time period and the sixth time period may be all the same, all different, or partially the same, and may be set according to actual situations, which is not particularly defined in the present application.

In the above-mentioned embodiment, the electrode 101 is controlled to be applied with the first electrical signal continuously, and the remaining electrodes 102-106 are controlled to be applied with the second electrical signal sequentially according to the set order, where the voltage of the second electrical signal applied to the remaining electrodes 102-106 is smaller than the voltage of the first electrical signal. As compared with applying the first electrical signal to all electrodes and switching the first electrical signal among all electrodes, in the embodiment of the present application, one electrode is applied with the first electrical signal, the remaining electrodes are applied with the second electrical signal, and the second electrical signal is switched among the remaining electrodes, so the power consumption is reduced, thereby reducing the power consumption of the electric field generating device and extending the standby duration of the battery used for power supply of the electric field generating device.

In some embodiments, m is 1, and controlling the first switch assembly to transmit the first electrical signal to m electrodes of the n electrodes, and controlling the second switch assembly to transmit the second electrical signal to at least two electrodes of the n-m electrodes, to generate the electric field between the electrodes with the first electrical signal and the electrodes with the second electrical signal includes:
controlling n first switch units to be turned on sequentially according to a set order, to enable n electrodes to receive the first electrical signal sequentially according to the set order, synchronously controlling n second switch units to be turned off sequentially according to the set order, and synchronously controlling second switch units combinations to be turned on sequentially according to the set order, to enable electrode combinations corresponding to the second switch units combinations to receive the second electrical signal sequentially according to the set order; where each second switch units combination includes at least two second switch units of n-1 second switch units that are not turned off, each electrode combination includes at least two electrodes that do not receive the first electrical signal.

The first switch assembly includes the n first switch units, and the second switch assembly includes the n second switch units.

In the above-mentioned embodiment, n electrodes are controlled to have the first electrical signal (high voltage signal) and the second electrical signal (low voltage signal) sequentially according to the set order, which further reduces the power consumption of the electric field generating device, thereby preventing the electric field generating device from overheating.

Optionally, still referring to Fig. 3, in order to prevent the electric field generating device from overheating, six first switch units 501 are controlled to be turned on sequentially according to the set order, so as to transmit the first electrical signal to the corresponding electrodes sequentially according to the set order. Six second switch units 601 electrically connected to the corresponding electrodes are synchronously controlled to be turned off sequentially according to the set order, and second switch units 601 that are not turned off are synchronously controlled to be turned on sequentially according to the set order, so as to transmit the second electrical signal to electrodes that do not receive the first electrical signal sequentially according to the set order.

Optionally, the set order includes: a clockwise order, a counterclockwise order, an n-pointed star shape order, or any other hop or non-continuous orders.

Optionally, a time interval for the second switch units 601 to be turned on or off sequentially according to the set order is not less than 20ms and not greater than 500ms.

Optionally, the time at which each electrode has the first electrical signal or the second electrical signal may be set according to actual situations, which is not particularly defined in the present application.

Thus, the electrodes 101-106 are applied with the first electrical signal and the second electrical signal sequentially according to the set order, which further reduces the power consumption of the electric field generating device, thereby preventing the electric field generating device from overheating.

Optionally, a time interval for the second switch units 601 to be turned on or off sequentially according to the set order is not less than 20ms and not greater than 500ms.

Note that the target biological tissue region includes the target biological tissue and a normal biological tissue, and the target biological tissue includes diseased cells, a tumor or a lesion, etc.

In order to further verify the technical effect of the present application, a case where the target biological tissue is a tumor is taken as an example, a two-dimensional model is performed on the target biological tissue and n electrodes surrounding the target biological tissue region, and a three-dimensional model is performed on a human thorax and a tumor in the human thorax.

### Two-dimensional model

A finite element modeling process is as follows.

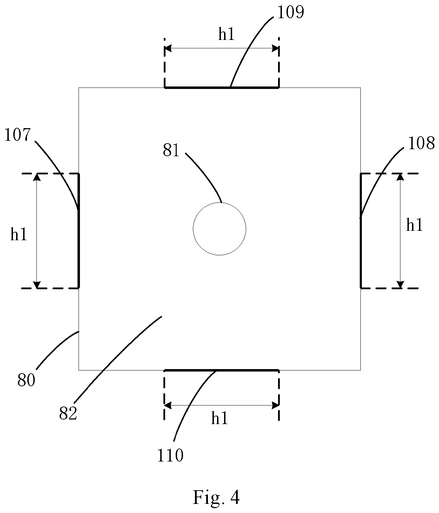

A geometric model is constructed in COMSOL. Next, a circle with a diameter of 40 mm (millimeters) is constructed as the tumor (i.e., the target biological tissue 81), a square with a side length of 200 mm is constructed as the target biological tissue region 80, where a center of the square coincides with a center of the circle of the tumor, and a region of the square outside the circle represents the normal biological tissue 82 outside of the tumor. Straight lines with a length of 80mm (h1 is 80mm in Fig. 4) are arranged respectively in the middle of the four edges of the above square as electrodes. The constructed model is shown in Fig. 4, and four electrodes are denoted as electrode 107, electrode 108, electrode 109 and electrode 110.

Material electrical parameter settings. The conductivity of a tumor region (i.e., the target biological tissue 81) is set to 0.24S/m and the dielectric constant thereof is set to 2000. The conductivity of a surrounding normal biological tissue 82 is set to 0.11S/m and the dielectric constant thereof is set to 1980.

Applied voltage waveform. In this embodiment, a continuous sine wave with a frequency of 150kHz (kilohertz) and a peak-to-peak value of 120V (volts) is applied, that is, the first electrical signal generating circuit is an AC signal generating circuit and outputs an AC voltage signal with a frequency of 150kHz (kilohertz) and a peak-to-peak value of 120V (volts).

Note that the first electrical signal outputted by the first electrical signal generating circuit is a high voltage signal, and the second electrical signal outputted by the second electrical signal generating circuit is a low voltage signal.

The electrode 107 is set as a power source receiving end, and configured to receive the first electrical signal outputted by the first electrical signal generating circuit, and the electrodes 108 to 110 are set as ground ends, and configured to receive the second electrical signal outputted by the second electrical signal generating circuit.

According to the above settings, following three cases are shown.
1. In a case where a single electrode is grounded, the electrode 107 is set to receive the first electrical signal (the high voltage signal) outputted by the first electrical signal generating circuit, and the electrode 108 is set to receive the second electrical signal (the low voltage signal) outputted by the second electrical signal generating circuit, that is, the electrode 107 receives a power source voltage, and the electrode 108 is grounded.

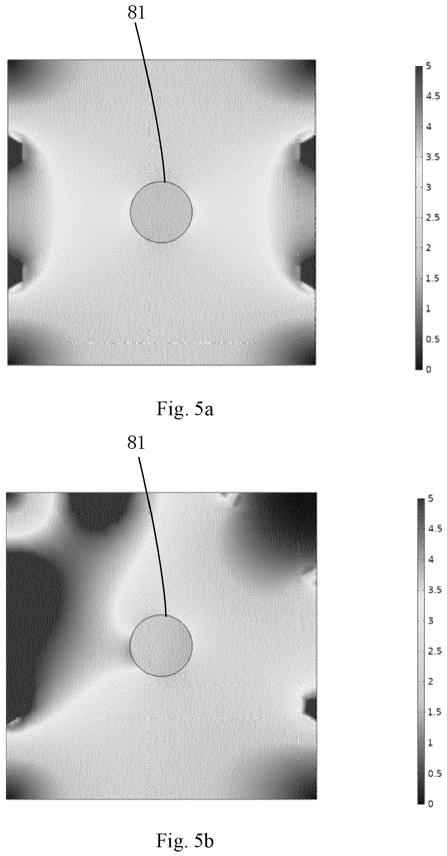

   Fig. 5a shows the case where the single electrode is grounded. After calculation, an average field strength in the tumor region is 1.6075V/cm (volts per centimeter).
2. In a first case where two electrodes are grounded, the electrode 107 is set to receive the first electrical signal (the high voltage signal) outputted by the first electrical signal generating circuit, and the electrode 108 as well as the electrode 109 are set to receive the second electrical signal (the low voltage signal) outputted by the second electrical signal generating circuit, that is, the electrode 107 receives a power source voltage, and the electrode 108 as well as the electrode 109 are grounded.
   Fig. 5b shows the case where two electrodes are grounded. After calculation, an average field strength in the tumor region is 1.6775V/cm. As compared with the case where the single electrode is grounded, the average field strength in the tumor region is increased by 4.35%.
3. In a second case where two electrodes are grounded, the electrode 107 is set to receive the first electrical signal (the high voltage signal) outputted by the first electrical signal generating circuit, and the electrode 108 as well as the electrode 110 are set to receive the second electrical signal (the low voltage signal) outputted by the second electrical signal generating circuit, that is, the electrode 107 receives a power source voltage, and the electrode 108 as well as the electrode 110 are grounded.

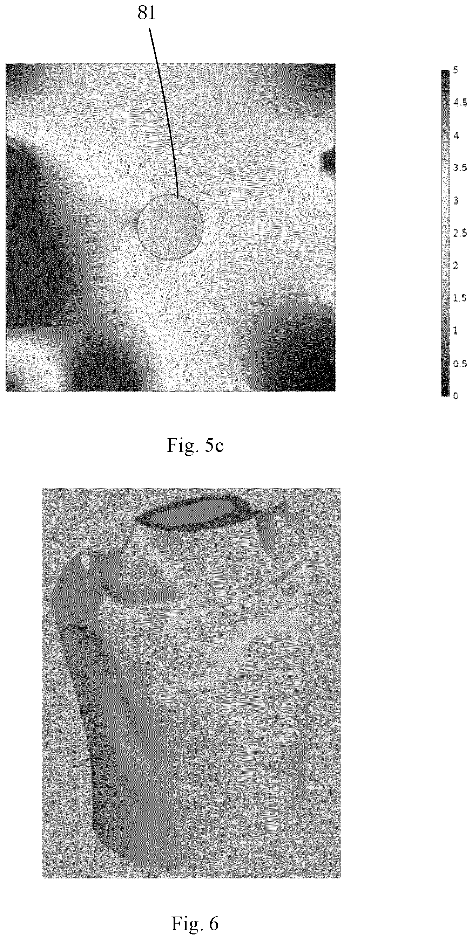

Fig. 5c shows the case where two electrodes are grounded. After calculation, an average field strength in the tumor region is 1.6775V/cm. As compared with the case where the single electrode is grounded, the average field strength in the tumor region is increased by 4.35%.

Average field strengths on the tumor in different cases are shown in Table 1.

**Table 1: Average field strengths on the tumor in different cases**

| | average field strength (V/cm) |
|---|---|
| case where the single electrode is grounded | 1.6075 |
| first case where two electrodes are grounded | 1.6775 |
| second case where two electrodes are grounded | 1.6775 |

Based on the above, when the quantity of electrodes with the high-voltage signal is maintained, the greater the quantity of electrodes with the low-voltage signal, the greater the average field strength in the tumor region.

In the embodiments of the present application, it is able to increase the coverage area of the electric field on the target biological tissue, increase the intensity of the electric field covering the target biological tissue region, and thereby increase the intensity of the electric field covering the target biological tissue, and improve the effect of inhibiting the division of such target biological tissue as diseased cells.

### Three-dimensional model

A finite element modeling process is as follows.

Construction of a human thorax. Approximate shapes of actual tissues are constructed in Rhino3D NURBS 7, and compared with multiple (Computed Tomography, CT) scanning, slices, so as to modify boundary contours, thereby ultimately restoring a realistic human body model. The modeling content includes skin, fat, muscles, bones, lungs, heart and liver. Some over-detailed tissues (such as connective tissues, pleurae) that have similar electrical parameters and are difficult to model are merged into muscle tissues. A final model is shown in Fig. 6.

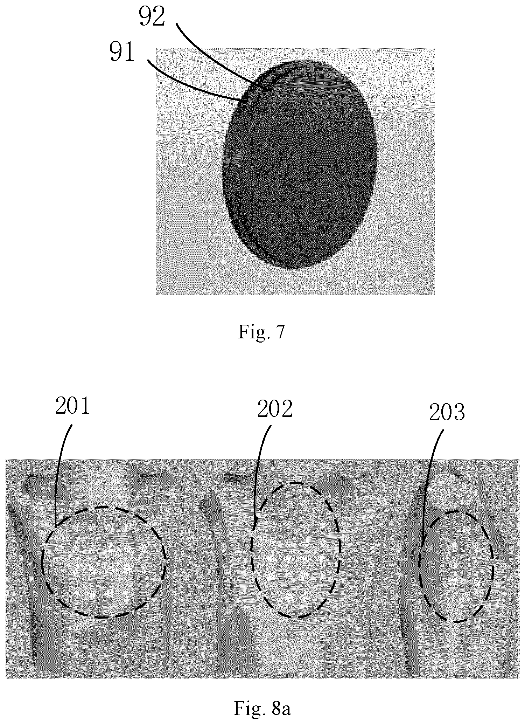

Construction of a single electrode. Each electrode is modeled as a double-layer structure as shown in Fig, 7 (i.e., a first layer 91 and a second layer 92 in Fig. 7), in which a layer having a large area (the first layer 91 in Fig. 7) that is close to a skin is a gel layer with a diameter of 21mm and a thickness ranging from 0.5 mm to 2mm, a layer having a small area (the second layer 92 in Fig. 7) is an insulation layer with a diameter of 20mm and a thickness of 1mm. The insulation layer is made of a ceramic material with high dielectric constant. The dielectric constant is close to 10,000. The electric field is transmitted to the human body through the ceramic layer and gel layer.

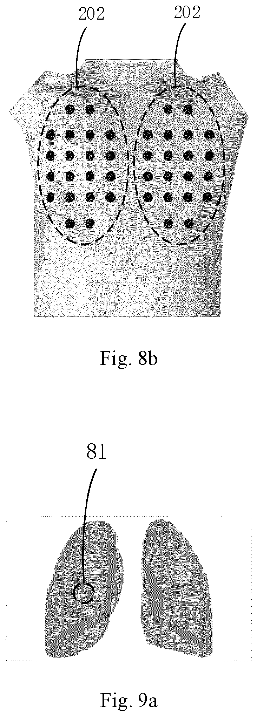

Construction of an electrode array. The electrode array is composed of multiple electrodes. For example, 5 electrodes, 10 electrodes, 15 electrodes, and other quantities of electrodes may form multiple different electrode arrays. Fig. 8a and Fig. 8b show the distribution of the electrode arrays applicable for human thoracic lung cancer treatment, where Fig. 8a shows the layout of an initial electrode array.

As shown in Fig. 8a, electrode arrays applicable for lung cancer treatment are categorized into three types, namely, a front electrode array (a first electrode array 201 located on the front side of the thorax of a human body in Fig. 8a), a back electrode array (a first electrode array 202 located on the back of the human body in Fig. 8a) and a side electrode array (a third electrode array 203 located on one side of the human body in Fig. 8a). Due to the large skin area on the front side of the thorax and the back of the human body, the front electrode array and the back electrode array each include more electrodes, for example, the front electrode array (i.e., the first electrode array 201) and the back electrode array (i.e., the second electrode array 202) each include 20 electrodes. Due to limited placement space on both sides of the human body, the electrode arrays positioned laterally each include fewer electrodes. Specifically, the third electrode array 203 including 13 electrodes is placed on each side of the human body. As shown in Fig. 8a, the first electrode array 201 is arranged on the front side of the thorax of the human body, the second electrode array 202 is arranged on the back of the human body, the third electrode array 203 is arranged on a left side of the human body, and the third electrode array 203 is arranged on a right side of the human body. That is, four electrode arrays (i.e., the first electrode array 201, the second electrode array 202, the third electrode array 203 and the third electrode array 203) are arranged on the front side of the thorax, back, left side, and right side of the human body.

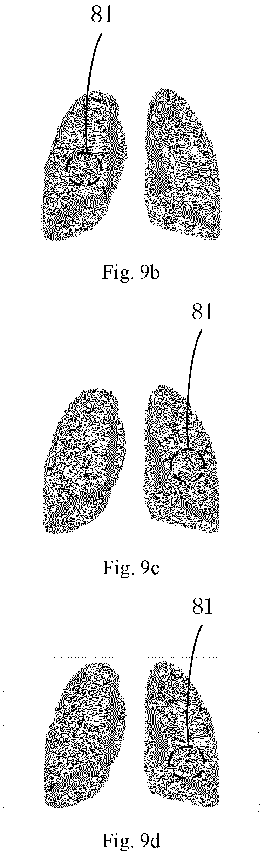

Construction of Tumor Models. A total of four virtual tumor models are created, as shown in Figs. 9a, 9b, 9c and 9d. In these figures, the target biological tissues 81 are assumed as Tumors T1, T2, T3, and T4. The sizes and locations of four tumors are indicated by dashed circles in Figs. 9a, 9b, 9c and 9d. In Fig. 9a, Tumor T1 in a middle lobe of a right lung has a diameter of 40mm. In Fig. 9b, Tumor T2 is in a middle lobe of the right lung. In Fig. 9c, Tumor T3 is in a middle lobe of a left lung. In Fig. 9d, Tumor T4 is in a lower lobe of a left lung. Tumors T2, T3 and T4 each have a diameter of 60mm.

After the model has been constructed, separate meshes for various tissues including electrodes, gels, skin, fat, bones, muscles, lungs, heart, liver and tumors are generated through mesh generation by using HyperMesh14.0, and then imported into COMSOL Multiphysics 5.5 one by one, so as to solve electrical quasi-static equations of Maxwell's equations by using a current module.

Material electrical parameter settings. As shown in Table 2, the conductivity and dielectric constant of each tissue are set to corresponding values.

**Table 2: conductivity and dielectric constant of each tissue**

| tissue | fat | muscle | bone | lung | heart | liver | tumor | ceramic | gel |
|---|---|---|---|---|---|---|---|---|---|
| conductivity (S/m) | 0.04 | 0.373 | 0.07 | 0.11 | 0.228 | 0.0954 | 0.24 | 0 | 0.1 |
| dielectric constant | 100 | 7110 | 350 | 1980 | 7333 | 6090 | 2000 | 10000 | 100 |

Simulation power source settings: Frequency domain simulation is used, a frequency thereof is set to 150kHz, and a normal current density on each electrode is set to 100mA/cm² (milliamps per square centimeter).

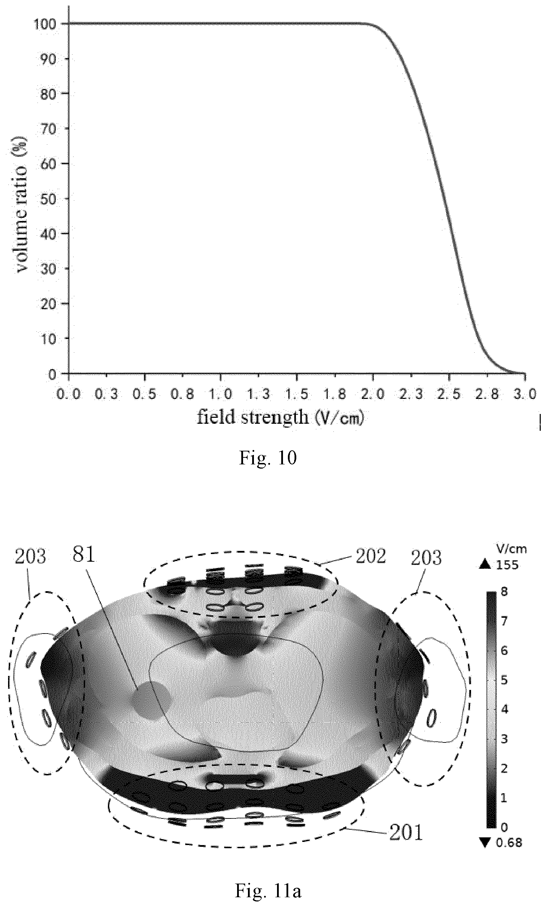

Field strength evaluation method: COMSOL is used to calculate a curve diagram between the electric field strength-volume (EVH) at the tumor, as shown in Fig. 10, where a horizontal axis is the field strength (V/cm), and a vertical axis is a volume ratio of the tumor (%). Each point on the curve represents a volume ratio of the tumor that is greater than a field strength value corresponding to the point. An area below the entire curve is used to represent the field strength coverage on the tumor, and the area can be obtained by integrating the curve. A value of the area is referred to as EAUC (Electric Field Area Under Curve).

Note that the first electrical signal outputted by the first electrical signal generating circuit is a high voltage signal, and the second electrical signal outputted by the second electrical signal generating circuit is a low voltage signal.

The front electrode array is set as a power source receiving end, and configured to receive the first electric signal outputted by the first signal generation circuit, and the back electrode array, the left side electrode array and the right side electrode array are set as ground ends, and configured to receive the second electric signal outputted by the second signal generation circuit.

In the case of Tumor T1 and Tumor T2, simulation results are shown in Table 3, where E_{AUC} values are compared. Two cases presented in Table 3 are as follows.
1. In a case where a single electrode array is grounded, the front electrode array (e.g., the first electrode array 201 in Fig. 8a) is set to receive the first electrical signal (the high voltage signal) outputted by the first electrical signal generating circuit, and the back electrode array (the second electrode array 202 in Fig. 8b) receives the second electrical signal (the low voltage signal) outputted by the second electrical signal generating circuit, that is, the front electrode array receives a power source voltage, and the back electrode array is grounded.
2. In a first case where two electrode arrays are grounded, the front electrode array (e.g., the first electrode array 201 in Fig. 8a) is set to receive the first electrical signal (the high voltage signal) outputted by the first electrical signal generating circuit, and the back electrode array (e.g., the second electrode array 202 in Fig. 8a) as well as the left side electrode array (e.g., the third electrode array 203 in Fig. 8a) receive the second electrical signal (the low voltage signal) outputted by the second electrical signal generating circuit, that is, the front electrode array receives a power source voltage, and the back electrode array as well as the left side electrode array are grounded.

As the results in Table 3 indicate, the E_{AUC} value in the case where two electrode arrays are grounded is higher than the E_{AUC} value in the case where the single electrode array is grounded, i.e., the E_{AUC} value is increased. It can be observed from Table 3 that, for Tumor T1, the E_{AUC} value in the case where two electrode arrays are grounded, as compared with the case where the single electrode array is grounded, is increased by 1.22%. For Tumor T2, the E_{AUC} value in the case where two electrode arrays are grounded, as compared with the case where the single electrode array is grounded, is increased by 1.23%.

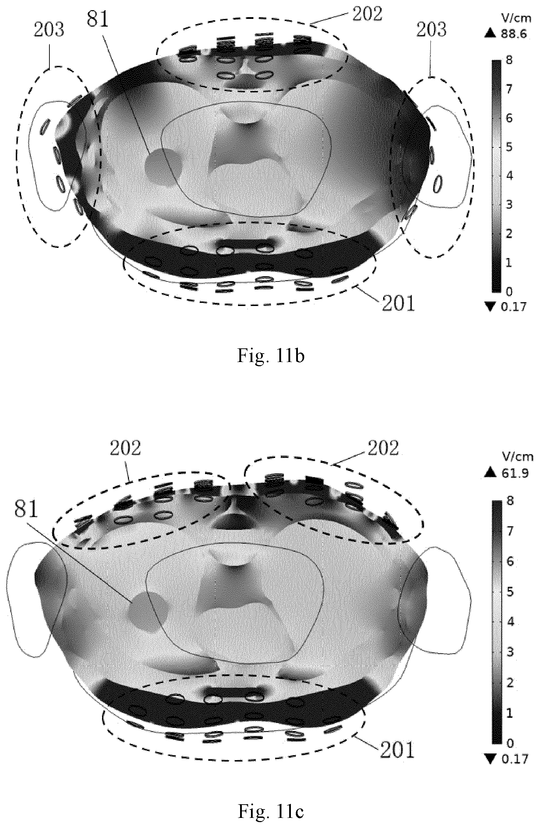

The distribution of the electric field in a horizontal plane of the thorax including Tumor T1 is shown in Fig. 11a and Fig. 11b, where a position of Tumor T1 is selected at the center of a tumor sphere. It may be observed from the figures that the intensity of the electric field located on the left side of the human body in Fig. 11b (e.g., a left part of Fig. 11b) is higher as compared with that on the left side of the human body in Fig. 11a.

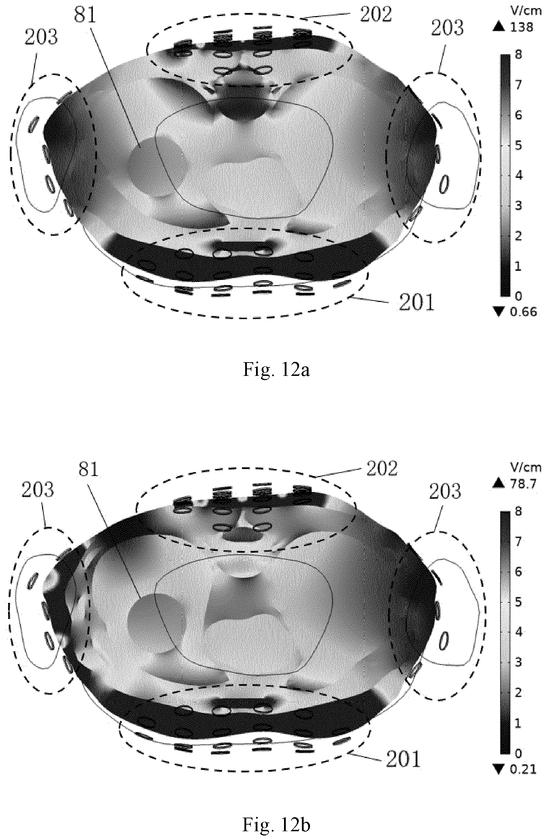

The distribution of the electric field in a horizontal plane of the thorax including Tumor T2 is shown in Fig. 12a and Fig. 12b, where a position of Tumor T2 is selected at the center of a tumor sphere. It may be observed from the figures that the intensity of the electric field located on the left side of the human body in Fig. 12b (e.g., a left part in the figure) is higher as compared with that on the left side in Fig. 12a.

**Table 3: E_{AUC} values at Tumor T1 and Tumor T2 in different cases**

| | E_{ACU} (V/cm) | |
|---|---|---|
| Tumor | T1 | T2 |
| case where the single electrode array (the back electrode array) is grounded | 2.43 | 2.45 |
| case where two electrode arrays (the back electrode array and the left side electrode array) are grounded | 2.46 | 2.48 |

In the case of Tumor T3 and Tumor T4, simulation results are shown in Table 4, where E_{AUC} values are compared. Two cases presented in Table 4 are as follows.
1. In a case where a single electrode array is grounded, the front electrode array (e.g., the first electrode array 201 in Fig. 8a) is set to receive the first electrical signal (the high voltage signal) outputted by the first electrical signal generating circuit, and the back electrode array (the second electrode array 202 in Fig. 8b) receives the second electrical signal (the low voltage signal) outputted by the second electrical signal generating circuit, that is, the front electrode array receives a power source voltage, and the back electrode array is grounded.
2. In a case where two electrode arrays are grounded, the front electrode array (e.g., the first electrode array 201 in Fig. 8a) is set to receive the first electrical signal (the high voltage signal) outputted by the first electrical signal generating circuit, and the back electrode array (e.g., the second electrode array 202 in Fig. 8a) as well as the left side electrode array (e.g., the third electrode array 203 in Fig. 8a) receive the second electrical signal (the low voltage signal) outputted by the second electrical signal generating circuit, that is, the front electrode array receives a power source voltage, and the back electrode array as well as the left side electrode array are grounded.

As shown in Table 4, the E_{AUC} value in the case where two electrode arrays are grounded is higher than the E_{AUC} value in the case where the single electrode array is grounded, i.e., the E_{AUC} value is increased. It can be observed from Table 3 that, for Tumor T3, the E_{AUC} value in the case where two electrode arrays are grounded, as compared with the case where the single electrode array is grounded, is increased by 16.20%. For Tumor T4, the E_{AUC} value in the case where two electrode arrays are grounded, as compared with the case where the single electrode array is grounded, is increased by 15.79%.

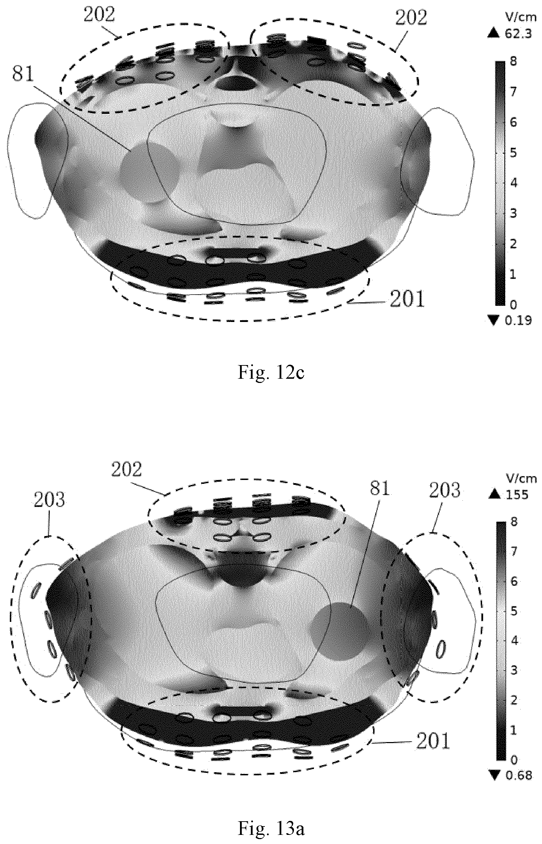

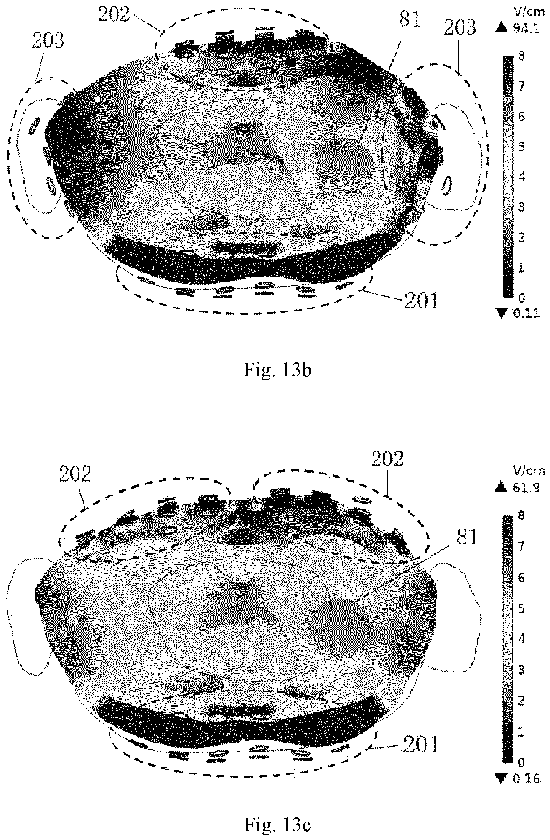

The distribution of the electric field in a horizontal plane of the thorax including Tumor T3 is shown in Fig. 13a and Fig. 13b, where a position of Tumor T3 is selected at the center of a tumor sphere. It may be observed from the figures that the intensity of the electric field located on the right side of the human body in Fig. 13b (e.g., a right part in the figure) is higher as compared with that on the right side of the human body in Fig. 13a.

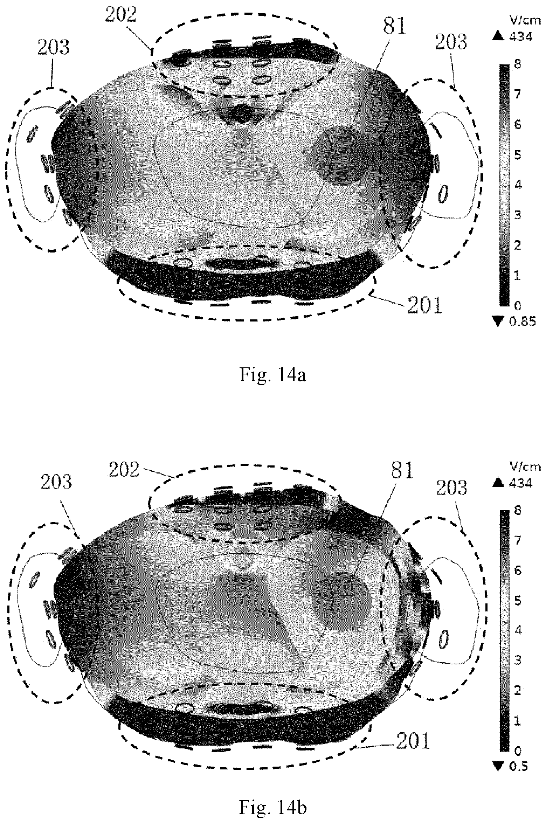

The distribution of the electric field in a horizontal plane of the thorax including Tumor T4 is shown in Fig. 14a and Fig. 14b, where a position of Tumor T4 is selected at the center of a tumor sphere. It may be observed from the figures that the intensity of the electric field located on the right side of the human body in Fig. 14b (e.g., a right part in the figure) is higher as compared with that on the right side of the human body in Fig. 14a.

**Table 4: E_{AUC} values at Tumor T3 and Tumor T4 in different cases**

| | E_{ACU} (V/cm) | |
|---|---|---|
| Tumor | T3 | T4 |
| case where the single electrode array (the back electrode array) is grounded | 1.79 | 2.09 |
| case where two electrode arrays (the back electrode array and the left side electrode array) are grounded | 2.08 | 2.42 |

From the above results, it may be found that when the case where the electrode array on the side close to the tumor and the back electrode array are grounded is used, as compared with the case where only the back electrode array is grounded, it is able to increase the field strength in the tumor region, increase the field strength coverage of the electric field generated between the electrode arrays, thereby to further improve the effect of inhibiting the division of such target biological tissue as diseased cells.

In addition, as shown in Fig. 8b, in the case of Tumor T1, Tumor T2, Tumor T3 and Tumor T4, two electrode arrays are placed at a left-of-center position and a right-of-center position of the back respectively. Two cases in presented Table 5 are as follows.
1. In a case where a single electrode array is grounded, the front electrode array (e.g., the first electrode array 201 in Fig. 8a) is set to receive the first electrical signal (the high voltage signal) outputted by the first electrical signal generating circuit, and the back electrode array (the second electrode array 202 in Fig. 8b) receives the second electrical signal (the low voltage signal) outputted by the second electrical signal generating circuit, that is, the front electrode array receives a power source voltage, and the back electrode array is grounded.
2. In a case where two electrode arrays are grounded, the front electrode array (e.g., the first electrode array 201 in Fig. 8a) is set to receive the first electrical signal (the high voltage signal) outputted by the first electrical signal generating circuit, and the back electrode arrays (e.g., two second electrode arrays 202 in Fig. 8b) receive the second electrical signal (the low voltage signal) outputted by the second electrical signal generating circuit, that is, the front electrode array receives a power source voltage, and the back electrode arrays are grounded.

It is observed that two second electrode arrays 202 with the low-voltage signal are arranged on the back, as compared with a case where only one second electrode array 202 with the low-voltage signal is arranged on the back, it is also able to increase the field strength at the tumor. Table 5 shows the comparison of E_{AUC} values at the four aforementioned tumor models between the case where one second electrode array 202 is arranged on the back and the case where two second electrode arrays 202 are arranged on the back.

From Table 5, it may be derived that, for Tumor T1, the E_{AUC} value is increased by 4.12% in the case where two electrode arrays are grounded, as compared with the case where the single electrode array is grounded. For Tumor T2, the E_{AUC} value is increased by 4.49% in the case where two electrode arrays are grounded, as compared with the case where the single electrode array is grounded. For Tumor T3, the E_{AUC} value is increased by 15.08% in the case where two electrode arrays are grounded, as compared with the case where the single electrode array is grounded. For Tumor T4, the E_{AUC} value is increased by 9.57% in the case where two electrode arrays are grounded, as compared with the case where the single electrode array is grounded.

The distribution of the electric field in a horizontal plane of the thorax including Tumor T1 is shown in Fig. 11a and Fig. 11c, where a position of Tumor T1 is selected at the center of a tumor sphere. It may be observed from the figures that the intensity of the electric field located on the back of the human body in Fig. 11c (e.g., in an upper part of Fig. 11c) is higher as compared with the intensity of the electric field in the upper part of Fig. 11a.

The distribution of the electric field in a horizontal plane of the thorax including Tumor T2 is shown in Fig. 12a and Fig. 12c, where a position of Tumor T2 is selected at the center of a tumor sphere. It may be observed from the figures that the intensity of the electric field located on the back of the human body in Fig. 12c (e.g., in an upper part of Fig. 12c) is higher as compared with the intensity of the electric field in the upper part of Fig. 12a.

The distribution of the electric field in a horizontal plane of the thorax including Tumor T3 is shown in Fig. 13a and Fig. 13c, where a position of Tumor T3 is selected at the center of a tumor sphere. It may be observed from the figures that the intensity of the electric field located on the back of the human body in Fig. 13c (e.g., in an upper part of Fig. 13c) is higher as compared with the intensity of the electric field in the upper part of Fig. 13a.

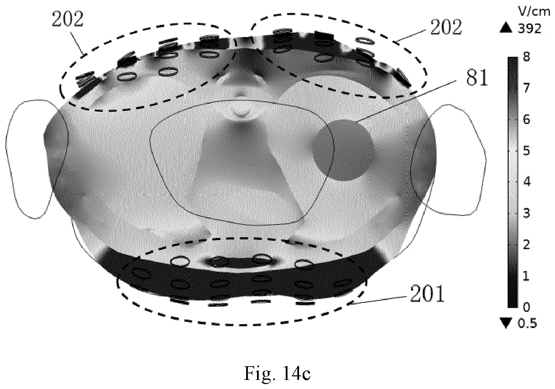

The distribution of the electric field in a horizontal plane of the thorax including Tumor T4 is shown in Fig. 14a and Fig. 14c, where a position of Tumor T4 is selected at the center of a tumor sphere. It may be observed from the figures that the intensity of the electric field located on the back of the human body in Fig. 14c (e.g., in an upper part of Fig. 14c) is higher as compared with the intensity of the electric field in the upper part of Fig. 14a.

**Table 5: E_{AUC} values at Tumor T1, Tumor T2, Tumor T3 and Tumor T4 in different cases**

| | EACU (V/cm) | | | |
|---|---|---|---|---|
| Tumor | T1 | T2 | T3 | T4 |
| case where the single electrode array (the back electrode array) is grounded | 2.43 | 2.45 | 1.79 | 2.09 |
| case where two electrode arrays (two electrode arrays on the back) are grounded | 2.53 | 2.56 | 2.06 | 2.29 |

Based on the above, when the quantity of electrodes with the high-voltage signal is maintained, as the quantity of electrodes with the low-voltage signal increases, the field strength coverage of the electric field generated between the electrode arrays is increased, thereby to further improve the effect of inhibiting the division of such target biological tissue as diseased cells.

In the embodiments of the present application, when the quantity of electrodes with the first electrical signal (high voltage signal) is maintained, the more the quantity of electrodes with the second electrical signal (low voltage signal), the more the electric fields generated between the electrodes with the first electrical signal and the electrodes with the second electrical signal, the larger the coverage area of the electric fields on the target biological tissue region, so as to increase the intensity of the electric field covering the target biological tissue region, thereby to increase the intensity of the electric field covering the target biological tissue, and further improve the effect of inhibiting the division of such target biological tissue as diseased cells.

Based on the same inventive concept, the embodiments of the present application provide a computer-readable storage medium having a computer program stored thereon, the computer program implements, when executed by a processor, the control method for the electric field generating device in any of the above optional embodiments of the present application.

The computer-readable storage medium in the embodiments of the present application is applicable for various optional embodiments of the control method for the electric field generating device. The computer-readable storage medium may be a non-volatile readable storage medium or a volatile readable storage medium, which is not particularly defined herein.

When the embodiments of the present application are implemented, at least the following beneficial effects can be achieved.
(1) In the electric field generating device of the embodiments of the present application, n electrodes surround the target biological tissue region in a set manner, and the electrical signal generator is controlled to apply the first electrical signal to m electrodes of the n electrodes, and apply the second electrical signal to at least two electrodes of the n-m electrodes, where n is an integer not less than 3, 1≤m<n, and m is an integer. In the embodiments of the present application, it is able to flexibly select the quantity of electrodes surrounding the target biological tissue region according to a position of the target biological tissue in the target biological tissue region, and control which electrodes to be applied to the second electrical signal, so that a coverage area of the electric field generated between the electrode with the first electrical signal and the electrodes with the second electrical signal best matches the position of the target biological tissue. Therefore, it is able to improve a matching degree between the coverage area of the electric field and the position of the target biological tissue, a coverage, and flexibility or adaptability of the electric field on the target biological tissue, thereby to increase the intensity of the electric field covering the target biological tissue, and further improve the effect of inhibiting the division of such target biological tissue as diseased cells.

Moreover, multiple electric fields can be generated between the electrode with the first electrical signal and at least two electrodes with the second electrical signal, and can be superimposed so as to enhance the intensity of an electric field at a superimposed region. When at least two electrodes with the second electrical signal are selected reasonably, it is able to improve the coverage of the electric field on the target biological tissue, thereby to improve the effect of inhibiting the division of such target biological tissue as diseased cells.
(2) In the above-mentioned embodiments of the present application, one of n electrodes is controlled to be applied with the first electrical signal continuously, and the remaining electrodes 102-106 are controlled to be applied with the second electrical signal sequentially according to the set order, where the voltage of the second electrical signal applied to the remaining electrodes 102-106 is smaller than the voltage of the first electrical signal. As compared with applying the first electrical signal to all electrodes and switching the first electrical signal among all electrodes, in the embodiment of the present application, one electrode is applied with the first electrical signal, the remaining electrodes are applied with the second electrical signal, and the second electrical signal is switched among the remaining electrodes, so the power consumption is reduced, thereby reducing the power consumption of the electric field generating device.
(3) In the above-mentioned embodiments of the present application, n electrodes are controlled to have the first electrical signal (high voltage signal) and the second electrical signal (low voltage signal) sequentially according to the set order, which further reduces the power consumption of the electric field generating device, thereby preventing the electric field generating device from overheating.
(4) When the quantity of electrodes with the first electrical signal (high voltage signal) is maintained, the more the quantity of electrodes with the second electrical signal (low voltage signal), the more the electric fields generated between the electrodes with the first electrical signal and the electrodes with the second electrical signal, the larger the coverage area of the electric fields on the target biological tissue region, so as to increase the intensity of the electric field covering the target biological tissue region, thereby to increase the intensity of the electric field covering the target biological tissue, and further improve the effect of inhibiting the division of such target biological tissue as diseased cells.

As can be appreciated by a person skilled in the art, steps, measures and schemes in various operations, methods and processes that have already been discussed in the embodiments of the present application may be replaced, modified, combined or deleted. In a possible embodiment of the present disclosure, the other steps, measures and schemes in various operations, methods and processes that have already been discussed in the embodiments of the present application may also be replaced, modified, rearranged, decomposed, combined or deleted. In another possible embodiment of the present application, steps, measures and schemes in various operations, methods and processes that are known in the related art and have already been discussed in the embodiments of the present application may also be replaced, modified, rearranged, decomposed, combined or deleted.

Such words as "first" and "second" are merely for illustrative purposes, rather than to implicitly or explicitly indicate the number of the defined technical features. In this regard, the technical features defined with such words as "first" and "second" may implicitly or explicitly include one or more technical features. Further, such a phrase as "a plurality of" is used to indicate that there are at least two, e.g., two or three, components, unless otherwise specified.

It should be further appreciated that, although with arrows, the steps in the flow charts may not be necessarily performed in an order indicated by the arrows. Unless otherwise defined, the order of the steps may not be strictly defined, i.e., the steps may also be performed in another order. In addition, each of at least parts of the steps in the flow charts may include a plurality of sub-steps or stages, and these sub-steps or stages may not be necessarily performed at the same time, i.e., they may also be performed at different times. Furthermore, these sub-steps or stages may not be necessarily performed sequentially, and instead, they may be performed alternately with the other steps or at least parts of sub-steps or stages of the other steps.

The above embodiments are partial embodiments of the present application, it should be appreciated that those skilled in the art may make various improvements and modifications without departing from the present disclosure, and theses improvement and modifications shall fall within the scope of the present disclosure, as defined by the appended claims.

## Claims

1. An electric field generating device, comprising
n electrodes (10), configured to surround a target biological tissue region in a set manner; where n is an integer not less than 3;
an electrical signal generator (25), electrically connected to the n electrodes (10);
a control signal generator (40), electrically connected to the electrical signal generator (25), and configured to control the electrical signal generator (25) to apply a first electrical signal to m electrodes of the n electrodes (10), and apply a second electrical signal to at least two electrodes of n-m electrodes, to generate an electric field between the electrodes with the first electrical signal and the electrodes with the second electrical signal; wherein a voltage of the second electrical signal is less than a voltage of the first electrical signal, 1≤m<n, and m is an integer;
wherein the quantity of electrodes with the first electrical signal is smaller than the quantity of electrodes with the second electrical signal.

2. The electric field generating device according to claim 1, wherein the electrical signal generator (25) comprises:
a first electrical signal generating circuit (20), electrically connected to the n electrodes (10), and configured to output the first electrical signal;
a second electrical signal generating circuit (30), electrically connected to the n electrodes (10) and configured to output the second electrical signal.

3. The electric field generating device according to claim 2, further comprising: a first switch assembly (50) and a second switch assembly (60) different from the first switch assembly (50);
wherein the first electrical signal generating circuit (20) is electrically connected to the n electrodes (10) through the first switch assembly (50), and the control signal generator (40) is electrically connected to the first switch assembly (50);
the second electrical signal generating circuit (30) is electrically connected to the n electrodes (10) through the second switch assembly (60), and the control signal generator (40) is electrically connected to the second switch assembly (60);
the control signal generator (40) is configured to control the first switch assembly (50) to transmit the first electrical signal to m electrodes of the n electrodes (10), and control the second switch assembly (60) to transmit the second electrical signal to at least two electrodes of n-m electrodes, to generate the electric field between the electrodes with the first electrical signal and the electrodes with the second electrical signal.

4. The electric field generating device according to claim 3, wherein the first switch assembly (50) comprises n first switch units (501); the second switch assembly (60) comprises n second switch units (601);
the n first switch units (501) are electrically connected to the n electrodes (10) respectively, and electrically connected to the first electrical signal generating circuit (20) and the control signal generator (40);
the n second switch units (601) are electrically connected to the n electrodes (10) respectively, and electrically connected to the second electrical signal generating circuit (30) and the control signal generator (40).

5. The electric field generating device according to claim 1, further comprising at least one of the following:
the first electrical signal comprising an Alternating Current (AC) voltage signal, a pulse voltage signal or a square wave voltage signal;
the second electrical signal comprising a constant voltage signal or a fluctuating voltage signal;
an absolute value of a voltage amplitude of the first electrical signal being not less than 0V and not greater than 500V;
an absolute value of a voltage amplitude of the second electrical signal being not less than 0 V and not greater than 10V;
an intensity of the electric field being not less than 0.1V/cm and not greater than 10V/cm;
a frequency of the electric field being not less than 50 kilohertz and not greater than 500 kilohertz;
m being 1.

6. The electric field generating device according to claim 5, wherein the absolute value of the voltage amplitude of the second electrical signal is 0V, 1V or 5V.

7. A control method for the electric field generating device according to any one of claims 1 to 6, **characterized by** comprising:
controlling the electrical signal generator (25) to apply a first electrical signal to m electrodes of the n electrodes (10), and apply a second electrical signal to at least two electrodes of n-m electrodes, to generate an electric field between the electrodes with the first electrical signal and the electrodes with the second electrical signal; where n is an integer not less than 3, 1≤m<n, m is an integer;
wherein the electric field generating device comprises the electrical signal generator (25) and the n electrodes (10) that are electrically connected to each other; a voltage of the second electrical signal is less than a voltage of the first electrical signal.

8. The control method for the electric field generating device according to claim 7, wherein controlling the electrical signal generator (25) to apply the first electrical signal to m electrodes of the n electrodes (10), and apply the second electrical signal to at least two electrodes of the n-m electrodes, to generate the electric field between the electrodes with the first electrical signal and the electrodes with the second electrical signal comprises:
controlling a first switch assembly (50) to transmit the first electrical signal to m electrodes of the n electrodes (10), and controlling a second switch assembly (60) to transmit the second electrical signal to at least two electrodes of the n-m electrodes, to generate the electric field between the electrodes with the first electrical signal and the electrodes with the second electrical signal;
wherein the electrical signal generator (25) comprises a first electrical signal generating circuit (20) and a second electrical signal generating circuit (30);
wherein the electric field generating device further comprises the first switch assembly (50) and the second switch assembly (60); the first switch assembly (50) is electrically connected to the first electrical signal generating circuit (20), the control signal generator (40) and n electrodes (10); the second switch assembly (60) is electrically connected to the second electrical signal generating circuit (30), the control signal generator (40) and n electrodes (10).

9. The control method for the electric field generating device according to claim 8, wherein controlling the first switch assembly (50) to transmit the first electrical signal to m electrodes of the n electrodes (10), and controlling the second switch assembly (60) to transmit the second electrical signal to at least two electrodes of the n-m electrodes, to generate the electric field between the electrodes with the first electrical signal and the electrodes with the second electrical signal comprises:
controlling a first one of first switch units (501) to transmit the first electrical signal to a first electrode of n electrodes (10), and controlling a second one of the first switch units (501) to a nth one of the first switch units (501) to be turned off; simultaneously controlling a first one of second switch units (601) that is electrically connected to the first electrode to be turned off, and controlling at least two second switch units (601) among a second one of the second switch units (601) to an nth one of the second switch units (601) to transmit the second electrical signal to at least two electrodes of n electrodes (10) except the first electrode;
wherein the first switch assembly (50) comprises n first switch units (501), and the second switch assembly (60) comprises n second switch units (601); the n first switch units (501) comprise the first one of the first switch units (501) to the nth one of the first switch units (501); the n second switch units (601) comprise the first one of the second switch units (601) to the nth one of the second switch units (601); the n electrodes (10) comprise the first electrode to an nth electrode.

10. The control method for the electric field generating device according to claim 8, wherein controlling the first switch assembly (50) to transmit the first electrical signal to m electrodes of the n electrodes (10), and controlling the second switch assembly (60) to transmit the second electrical signal to at least two electrodes of the n-m electrodes, to generate the electric field between the electrodes with the first electrical signal and the electrodes with the second electrical signal comprises:
controlling a first one of first switch units (501) to transmit the first electrical signal to a first electrode of n electrodes (10), and controlling a second one of the first switch units (501) to an nth one of the first switch units (501) to be turned off; simultaneously controlling a first one of second switch units (601) that is electrically connected to the first electrode to be turned off, and synchronously controlling at least two second switch units (601) among a second one of the second switch units (601) to an nth one of the second switch units (601) to be turned on sequentially according to a set order, to transmit the second electrical signal to at least two electrodes of n electrodes (10) except the first electrode according to the set order.

11. The control method for the electric field generating device according to claim 8, wherein m is 1, and controlling the first switch assembly (50) to transmit the first electrical signal to m electrodes of the n electrodes (10), and controlling the second switch assembly (60) to transmit the second electrical signal to at least two electrodes of the n-m electrodes, to generate the electric field between the electrodes with the first electrical signal and the electrodes with the second electrical signal comprises:
controlling n first switch units (501) to be turned on sequentially according to a set order, to enable n electrodes (10) to receive the first electrical signal sequentially according to the set order, synchronously controlling n second switch units (601) to be turned off sequentially according to the set order, and synchronously controlling second switch units (601) combinations to be turned on sequentially according to the set order, to enable electrode combinations corresponding to the second switch units (601) combinations to receive the second electrical signal sequentially according to the set order; wherein each second switch units combination comprises at least two second switch units (601) of n-1 second switch units (601) that are not turned off; each electrode combination comprises at least two electrodes that do not receive the first electrical signal;
wherein the first switch assembly (50) comprises the n first switch units (501), and the second switch assembly (60) comprises the n second switch units (601).

12. The control method for the electric field generating device according to claim 10 or 11, wherein a time interval for the second switch units (601) to be turned on or off sequentially according to the set order is not less than 20 milliseconds and not greater than 500 milliseconds.

13. The control method for the electric field generating device according to claim 7, wherein the first electrical signal applied by the electrical signal generator (25) to m electrodes of n electrodes (10) is a high voltage signal, and the second electrical signal applied by the electrical signal generator (25) to at least two electrodes of n-m electrodes is controlled to be a low voltage signal.

14. A computer-readable storage medium having a computer program stored thereon, **characterized by** the computer program implementing, when executed by a processor, the control method for the electric field generating device according to any one of claims 7 to 13.

## Patentansprüche

1. Vorrichtung zur Erzeugung eines elektrischen Feldes, umfassend
n Elektroden (10), so konfiguriert, dass sie eine Zielregion biologischen Gewebes in einer festgelegten Weise umgeben; wobei n eine ganze Zahl nicht kleiner als 3 ist;
einen elektrischen Signalgeber (25), der elektrisch mit den n Elektroden (10) verbunden ist;
einen Steuersignalgeber (40), der elektrisch mit dem elektrischen Signalgeber (25) verbunden ist und so konfiguriert ist, dass er den elektrischen Signalgeber (25) so steuert, dass ein erstes elektrisches Signal an m Elektroden der n Elektroden (10) angelegt wird und ein zweites elektrisches Signal an mindestens zwei Elektroden von n-m Elektroden anzulegen, um ein elektrisches Feld zwischen den Elektroden mit dem ersten elektrischen Signal und den Elektroden mit dem zweiten elektrischen Signal zu erzeugen; wobei eine Spannung des zweiten elektrischen Signals geringer ist als eine Spannung des ersten elektrischen Signals, wobei 1≤m<n ist und m eine ganze Zahl ist;
wobei die Anzahl der Elektroden mit dem ersten elektrischen Signal kleiner ist als die Anzahl der Elektroden mit dem zweiten elektrischen Signal.

2. Vorrichtung zur Erzeugung eines elektrischen Feldes nach Anspruch 1, wobei der elektrische Signalgeber (25) umfasst:
eine erste Schaltung zur Erzeugung des elektrischen Signals (20), die elektrisch mit den n Elektroden (10) verbunden ist und so konfiguriert ist, dass sie das erste elektrische Signal ausgibt;
eine zweite Schaltung zur Erzeugung des elektrischen Signals (30), die elektrisch mit den n Elektroden (10) verbunden ist und so konfiguriert ist, dass sie das zweite elektrische Signal ausgibt.

3. Vorrichtung zur Erzeugung eines elektrischen Feldes nach Anspruch 2, ferner umfassend: eine erste Schaltbaugruppe (50) und eine zweite Schaltbaugruppe (60), die von der ersten Schaltbaugruppe (50) verschieden ist; zur Erzeugung des elektrischen Signals (20) über die erste Schaltbaugruppe (50) mit den n Elektroden (10) elektrisch verbunden ist und der Steuersignalgeber (40) mit der ersten Schaltbaugruppe (50) elektrisch verbunden ist;
die zweite Schaltung zur Erzeugung des elektrischen Signals (30) über die zweite Schaltbaugruppe (60) mit den n Elektroden (10) elektrisch verbunden ist und der Steuersignalgeber (40) mit der zweiten Schaltbaugruppe (60) elektrisch verbunden ist;
der Steuersignalgeber (40) so konfiguriert ist, dass er die erste Schaltbaugruppe (50) so steuert, dass sie das erste elektrische Signal an m Elektroden der n Elektroden (10) überträgt, und die zweite Schaltbaugruppe (60) so steuert, dass sie das zweite elektrische Signal an mindestens zwei Elektroden von n-m Elektroden überträgt, um das elektrische Feld zwischen den Elektroden mit dem ersten elektrischen Signal und den Elektroden mit dem zweiten elektrischen Signal zu erzeugen.

4. Vorrichtung zur Erzeugung eines elektrischen Feldes gemäß Anspruch 3, wobei die erste Schaltbaugruppe (50) n erste Schalteinheiten (501) umfasst; die zweite Schaltbaugruppe (60) n zweite Schalteinheiten (601) umfasst;
die n ersten Schalteinheiten (501) jeweils elektrisch mit den n Elektroden (10) verbunden sind sowie elektrisch mit der ersten Schaltung zur Erzeugung des elektrischen Signals (20) und dem Steuersignalgeber (40) verbunden sind;
die n zweiten Schalteinheiten (601) jeweils elektrisch mit den n Elektroden (10) verbunden sind sowie elektrisch mit der zweiten Schaltung zur Erzeugung des elektrischen Signals (30) und dem Steuersignalgeber (40) verbunden sind.

5. Vorrichtung zur Erzeugung eines elektrischen Feldes nach Anspruch 1, die ferner mindestens eines der folgenden umfasst:
das erste elektrische Signal umfassend ein Wechselspannungssignal (AC), ein Impulsspannungssignal oder ein Rechteckwellenspannungssignal;
das zweite elektrische Signal umfassend ein Konstantspannungssignal oder ein schwankendes Spannungssignal;
ein Absolutwert einer Spannungsamplitude des ersten elektrischen Signals nicht weniger als 0 V und nicht mehr als 500 V beträgt;
ein Absolutwert einer Spannungsamplitude des zweiten elektrischen Signals nicht weniger als 0 V und nicht mehr als 10 V beträgt;
eine Intensität des elektrischen Feldes nicht weniger als 0,1 V/cm und nicht mehr als 10 V/cm beträgt;
eine Frequenz des elektrischen Feldes nicht weniger als 50 Kilohertz und nicht mehr als 500 Kilohertz beträgt;
m 1 ist.

6. Vorrichtung zur Erzeugung eines elektrischen Feldes gemäß Anspruch 5, wobei der Absolutwert der Spannungsamplitude des zweiten elektrischen Signals 0 V, 1 V oder 5 V beträgt.

7. Steuerungsverfahren für die Vorrichtung zur Erzeugung eines elektrischen Feldes gemäß einem der Ansprüche 1 bis 6, **dadurch gekennzeichnet, dass** sie umfassen:
Steuern des elektrischen Signalgebers (25), um ein erstes elektrisches Signal an die m Elektroden der n Elektroden (10) anzulegen und ein zweites elektrisches Signal an mindestens zwei Elektroden von n-m Elektroden anzulegen, um ein elektrisches Feld zwischen den Elektroden mit dem ersten elektrischen Signal und den Elektroden mit dem zweiten elektrischen Signal zu erzeugen; wobei n eine ganze Zahl ist, die nicht kleiner als 3 ist, 1≤m<n, m eine ganze Zahl ist;
wobei die Vorrichtung zur Erzeugung eines elektrischen Feldes den elektrischen Signalgeber (25) und die n Elektroden (10) umfasst, die elektrisch miteinander verbunden sind; eine Spannung des zweiten elektrischen Signals geringer ist als eine Spannung des ersten elektrischen Signals.

8. Steuerungsverfahren für die Vorrichtung zur Erzeugung eines elektrischen Feldes gemäß Anspruch 7, wobei die Steuerung des elektrischen Signalgebers (25) zum Anlegen des ersten elektrischen Signals an m Elektroden der n Elektroden (10) und zum Anlegen des zweiten elektrischen Signals an mindestens zwei Elektroden der n-m Elektroden, zum Erzeugen des elektrischen Feldes zwischen den Elektroden mit dem ersten elektrischen Signal und den Elektroden mit dem zweiten elektrischen Signal, umfassen:
Steuern einer ersten Schaltbaugruppe (50) zum Übertragen des ersten elektrischen Signals an m Elektroden der n Elektroden (10), und Steuern einer zweiten Schaltbaugruppe (60) zum Übertragen des zweiten elektrischen Signals an mindestens zwei Elektroden der n-m Elektroden, zum Erzeugen des elektrische Feld zwischen den Elektroden mit dem ersten elektrischen Signal und den Elektroden mit dem zweiten elektrischen Signal;
wobei der elektrische Signalgeber (25) eine erste Schaltung zur Erzeugung des elektrischen Signals (20) und eine zweite Schaltung zur Erzeugung des elektrischen Signals (30) umfasst;
wobei die Vorrichtung zum Erzeugen eines elektrischen Feldes ferner die erste Schaltbaugruppe (50) und die zweite Schaltbaugruppe (60) umfasst; die erste Schaltbaugruppe (50) elektrisch mit der ersten Schaltung zur Erzeugung des elektrischen Signals (20), dem Steuersignalgeber (40) und n Elektroden (10) elektrisch verbunden ist; die zweite Schaltbaugruppe (60) mit der zweiten Schaltung zur Erzeugung des elektrischen Signals (30), dem Steuersignalgeber (40) und n Elektroden (10) elektrisch verbunden ist.

9. Steuerungsverfahren für die Vorrichtung zur Erzeugung eines elektrischen Feldes gemäß Anspruch 8, wobei das Steuern der ersten Schaltbaugruppe (50), um das erste elektrische Signal an m Elektroden der n Elektroden (10) zu übertragen, und das Steuern der zweiten Schaltbaugruppe (60), um das zweite elektrische Signal an mindestens zwei Elektroden der n-m Elektroden zu übertragen, um das elektrische Feld zwischen den Elektroden mit dem ersten elektrischen Signal und den Elektroden mit dem zweiten elektrischen Signal zu erzeugen, umfasst:
Steuern einer ersten der ersten Schalteinheiten (501), um das erste elektrische Signal zu einer ersten Elektrode von n Elektroden (10) zu übertragen, und Steuern einer zweiten von den ersten Schalteinheiten (501) zu einer n-ten von den ersten Schalteinheiten (501), um ausgeschaltet zu werden; gleichzeitiges Steuern einer ersten von den zweiten Schalteinheiten (601), die elektrisch mit der ersten Elektrode verbunden ist, die ausgeschaltet werden soll, und Steuern von mindestens zwei zweiten Schalteinheiten (601) von einer zweiten der zweiten Schalteinheiten (601) zu einer n-ten der zweiten Schalteinheiten (601), um das zweites elektrisches Signal an mindestens zwei Elektroden von n Elektroden (10) mit Ausnahme der ersten Elektrode zu übertragen,
wobei die erste Schaltbaugruppe (50) n erste Schalteinheiten (501) umfasst und die zweite Schaltbaugruppe (60) n zweite Schalteinheiten (601) umfasst; die n ersten Schalteinheiten 5 (501) die erste der ersten Schalteinheiten (501) bis zur n-ten der ersten Schalteinheiten (501) umfassen die n zweiten Schalteinheiten (601) umfassen die erste der zweiten Schalteinheiten (601) bis zur n-ten der zweiten Schalteinheiten (601); die n Elektroden (10) umfassen die erste Elektrode bis zu einer n-ten Elektrode.

10. Steuerungsverfahren für die Vorrichtung zur Erzeugung eines elektrischen Feldes gemäß Anspruch 8, wobei das Steuern der ersten Schaltbaugruppe (50) um das erste elektrische Signal an m Elektroden der n Elektroden (10) zu übertragen, und das Steuern der zweiten Schaltbaugruppe (60) um das zweite elektrische Signal an mindestens zwei Elektroden der n-m Elektroden zu übertragen, um das elektrische Feld zwischen den Elektroden mit dem ersten elektrischen Signal und den Elektroden mit dem zweiten elektrischen Signal zu erzeugen, umfasst:
Steuern einer ersten der ersten Schalteinheiten (501), um das erste elektrische Signal zu einer ersten Elektrode von n Elektroden (10) zu übertragen, und Steuern einer zweiten von den ersten Schalteinheiten (501) zu einer n-ten von den ersten Schalteinheiten (501), um ausgeschaltet zu werden, gleichzeitiges Steuern einer ersten von den zweiten Schalteinheiten (601), die elektrisch mit der ersten Elektrode verbunden ist, die ausgeschaltet werden soll, und synchrones Steuern von mindestens zwei zweiten Schalteinheiten (601) unter einer zweiten der zweiten Schalteinheiten (601) bis zu einer n-ten der zweiten Schalteinheiten (601), die sequentiell gemäß einer festgelegten Reihenfolge eingeschaltet werden sollen, um das zweite elektrische Signal gemäß der festgelegten Reihenfolge an mindestens zwei Elektroden von n Elektroden (10) mit Ausnahme der ersten Elektrode zu übertragen.

11. Steuerungsverfahren für die Vorrichtung zur Erzeugung eines elektrischen Feldes gemäß Anspruch 8 wobei m 1 ist, und Steuern der ersten Schaltbaugruppe (50), um das erste elektrische Signal an m Elektroden der n Elektroden (10) zu übertragen, und Steuern der zweiten Schaltbaugruppe (60), um das zweite elektrische Signal an mindestens zwei Elektroden der n-m Elektroden zu übertragen, um das elektrische Feld zwischen den Elektroden mit dem ersten elektrischen Signal und den Elektroden mit dem zweiten elektrischen Signal zu erzeugen, umfasst:
Steuern von n ersten Schalteinheiten (501), die sequentiell gemäß einer festgelegten Reihenfolge eingeschaltet werden sollen, damit n Elektroden (10) das erste elektrische Signal sequentiell gemäß der festgelegten Reihenfolge empfangen können, synchrones Steuern von n zweiten Schalteinheiten (601), die sequentiell entsprechend der festgelegten Reihenfolge ausgeschaltet werden sollen, und synchrones Steuern von Kombinationen aus zweiten Schalteinheiten (601), die sequentiell entsprechend der festgelegten Reihenfolge eingeschaltet werden sollen, um Elektrodenkombinationen, die den Kombinationen aus zweiten Schalteinheiten (601) entsprechen, zu ermöglichen, das zweite elektrische Signal sequentiell entsprechend der festgelegten Reihenfolge zu empfangen; wobei jede Kombination aus zweiten Schalteinheiten mindestens zwei zweite Schalteinheiten (601) von n-1 zweiten Schalteinheiten (601) umfasst, die nicht ausgeschaltet sind; jede Elektrodenkombination mindestens zwei Elektroden umfasst, die das erste elektrische Signal nicht empfangen;
wobei die erste Schaltbaugruppe (50) die n ersten Schalteinheiten (501) umfasst und die zweite Schaltbaugruppe (60) die n zweiten Schalteinheiten (601) umfasst.

12. Steuerungsverfahren für die Vorrichtung zur Erzeugung eines elektrischen Feldes gemäß Anspruch 10 oder 11, wobei ein Zeitintervall, in dem die zweiten Schalteinheiten (601) sequentiell gemäß der festgelegten Reihenfolge ein- oder ausgeschaltet werden, nicht weniger als 20 Millisekunden und nicht mehr als 500 Millisekunden beträgt.

13. Steuerungsverfahren für die Vorrichtung zur Erzeugung eines elektrischen Feldes gemäß Anspruch 7, wobei das erste elektrische Signal, das vom elektrischen Signalgeber (25) an m Elektroden von n Elektroden (10) angelegt wird, ein Hochspannungssignal ist, und das zweite elektrische Signal, das vom elektrischen Signalgeber (25) an mindestens zwei Elektroden von n-m Elektroden angelegt wird, so gesteuert wird, dass es ein Niederspannungssignal ist.

14. Computerlesbares Speichermedium, das ein darauf gespeichertes Computerprogramm aufweist, **dadurch gekennzeichnet, dass** das Computerprogramm bei Ausführung durch einen Prozessor das Steuerverfahren für die Vorrichtung zur Erzeugung eines elektrischen Feldes gemäß einem der Ansprüche 7 bis 13 implementiert.

## Revendications

1. Dispositif générateur de champ électrique, comprenant
n électrodes (10), conçues pour entourer une région de tissu biologique cible d'une manière définie ; dans lequel n est un nombre entier non inférieur à 3 ;
un générateur de signaux électriques (25), connecté électriquement aux n électrodes (10) ;
un générateur de signaux de commande (40), connecté électriquement au générateur de signaux électriques (25) et conçu pour commander le générateur de signaux électriques (25) pour appliquer un premier signal électrique à m électrodes des n électrodes (10), et pour appliquer un deuxième signal électrique à au moins deux électrodes de n-m électrodes, afin de générer un champ électrique entre les électrodes avec le premier signal électrique et les électrodes avec le deuxième signal électrique, une tension du deuxième signal électrique étant inférieure à une tension du premier signal électrique, I≤m<n, et m étant un nombre entier ;
dans lequel la quantité d'électrodes avec le premier signal électrique est inférieure à la quantité d'électrodes avec le deuxième signal électrique.

2. Dispositif générateur de champ électrique selon la revendication 1, dans lequel le générateur de signaux électriques (25) comprend :
un premier circuit générateur de signaux électriques (20), connecté électriquement aux n électrodes (10), et conçu pour émettre le premier signal électrique ;
un deuxième circuit générateur de signaux électriques (30), connecté électriquement aux n électrodes (10), et conçu pour émettre le deuxième signal électrique.

3. Dispositif générateur de champ électrique selon la revendication 2, comprenant en outre : un premier ensemble commutateur (50) et un deuxième ensemble commutateur (60) différent du premier ensemble commutateur (50) ;
dans lequel le premier circuit générateur de signaux électriques (20) est connecté électriquement aux n électrodes (10) à travers le premier ensemble commutateur (50), et le générateur de signaux de commande (40) est connecté électriquement au premier ensemble commutateur (50) ;
le deuxième circuit générateur de signaux électriques (30) est connecté électriquement aux n électrodes (10) à travers le deuxième ensemble commutateur (60), et le générateur de signaux de commande (40) est connecté électriquement au deuxième ensemble commutateur (60) ;
le générateur de signaux de commande (40) est conçu pour commander le premier ensemble commutateur (50) pour transmettre le premier signal électrique à m électrodes des n électrodes (10), et pour commander le deuxième ensemble commutateur (60) pour transmettre le deuxième signal électrique à au moins deux électrodes de n-m électrodes, afin de générer le champ électrique entre les électrodes avec le premier signal électrique et les électrodes avec le deuxième signal électrique.

4. Dispositif générateur de champ électrique selon la revendication 3, dans lequel le premier ensemble commutateur (50) comprend n premières unités de commutation (501) ; le deuxième ensemble commutateur (60) comprend n deuxièmes unités de commutation (601) ;
les n premières unités de commutation (501) sont connectées électriquement aux n électrodes (10) respectivement, et connectées électriquement au premier circuit générateur de signaux électriques (20) et au générateur de signaux de commande (40) ;
les n deuxièmes unités de commutation (601) sont connectées électriquement aux n électrodes (10) respectivement, et connectées électriquement au deuxième circuit générateur de signaux électriques (30) et au générateur de signaux de commande (40).

5. Dispositif générateur de champ électrique selon la revendication 1, comprenant en outre au moins une des suivantes propriétés :
le premier signal électrique comprenant un signal de tension de courant alternatif (CA), un signal de tension d'impulsion ou un signal de tension à onde carrée ;
le deuxième signal électrique comprenant un signal de tension constante ou un signal de tension fluctuante ;
une valeur absolue d'une amplitude de tension du premier signal électrique étant non inférieure à 0 V et non supérieure à 500 V ;
une valeur absolue d'une amplitude de tension du deuxième signal électrique étant non inférieure à 0 V et non supérieure à 10 V ;
une intensité du champ électrique étant non inférieure à 0,1 V/cm et non supérieure à 10 V/cm ;
une fréquence du champ électrique étant non inférieure à 50 kilohertz et non supérieure à 500 kilohertz ;
m étant 1.

6. Dispositif générateur de champ électrique selon la revendication 5, dans lequel la valeur absolue de l'amplitude de tension du deuxième signal électrique est de 0 V, 1 V ou 5 V.

7. Procédé de commande pour le dispositif générateur de champ électrique selon l'une quelconque des revendications 1 à 6, **caractérisé par le fait qu'**il comprend :
la commande du générateur de signaux électriques (25) pour appliquer un premier signal électrique à m électrodes des n électrodes (10), et pour appliquer un deuxième signal électrique à au moins deux électrodes des n-m électrodes, afin de générer un champ électrique entre les électrodes avec le premier signal électrique et les électrodes avec le deuxième signal électrique ; dans lequel n est un nombre entier non inférieur à 3, 1≤m<n, m est un nombre entier ;
dans lequel le dispositif générateur de champ électrique comprend le générateur de signaux électriques (25) et les n électrodes (10) qui sont connectées électriquement les unes aux autres ; une tension du deuxième signal électrique est inférieure à une tension du premier signal électrique.

8. Procédé de commande pour le dispositif générateur de champ électrique selon la revendication 7, dans lequel la commande du générateur de signaux électriques (25) pour appliquer le premier signal électrique à m électrodes des n électrodes (10), et pour appliquer le deuxième signal électrique à au moins deux électrodes des n-m électrodes, afin de générer le champ électrique entre les électrodes avec le premier signal électrique et les électrodes avec le deuxième signal électrique comprend :
la commande d'un premier ensemble commutateur (50) pour transmettre le premier signal électrique à m électrodes des n électrodes (10), et la commande d'un deuxième ensemble commutateur (60) pour transmettre le deuxième signal électrique à au moins deux électrodes des n-m électrodes, afin de générer le champ électrique entre les électrodes avec le premier signal électrique et les électrodes avec le deuxième signal électrique ;
dans lequel le générateur de signaux électriques (25) comprend un premier circuit générateur de signaux électriques (20) et un deuxième circuit générateur de signaux électriques (30) ;
dans lequel le dispositif générateur de champ électrique comprend en outre le premier ensemble commutateur (50) et le deuxième ensemble commutateur (60) ; le premier ensemble commutateur (50) est connecté électriquement au premier circuit générateur de signaux électriques (20), au générateur de signaux de commande (40) et à n électrodes (10) ; le deuxième ensemble commutateur (60) est connecté électriquement au deuxième circuit générateur de signaux électriques (30), au générateur de signaux de commande (40) et à n électrodes (10).

9. Procédé de commande pour le dispositif générateur de champ électrique selon la revendication 8, dans lequel la commande du premier ensemble commutateur (50) pour transmettre le premier signal électrique à m électrodes des n électrodes (10), et la commande du deuxième ensemble commutateur (60) pour transmettre le deuxième signal électrique à au moins deux électrodes des n-m électrodes, afin de générer le champ électrique entre les électrodes avec le premier signal électrique et les électrodes avec le deuxième signal électrique comprend :
la commande d'une première de premières unités de commutation (501) pour transmettre le premier signal électrique à une première electrode de n électrodes (10), et la commande d'une deuxième des premières unités de commutation (501) à une nième des premières unités de commutation (501) pour être désactivées ; simultanément la commande d'une première de deuxièmes unités de commutation (601) qui est connectée électriquement à la premier electrode pour être désactivée, et la commande d'au moins deux deuxièmes unités de commutation (601) parmi une deuxième des deuxièmes unités de commutation (601) à une nième des deuxièmes unités de commutation (601) pour transmettre le deuxième signal électrique à au moins deux électrodes de n électrodes (10) excepté la première electrode ;
dans lequel le premier ensemble commutateur (50) comprend n premières unités de commutation (501), et le deuxième ensemble commutateur (60) comprend n deuxièmes unités de commutation (601) ; les n premières unités de commutation (501) comprennent la première des premières unités de commutation (501) à la nième des premières unités de commutation (501) ; les n deuxièmes unités de commutation (601) comprennent la première des deuxièmes unités de commutation (601) à la nième des deuxièmes unités de commutation (601) ; les n électrodes (10) comprennent la première electrode à la nième electrode.

10. Procédé de commande pour le dispositif générateur de champ électrique selon la revendication 8, dans lequel la commande du premier ensemble commutateur (50) pour transmettre le premier signal électrique à m électrodes des n électrodes (10), et la commande du deuxième ensemble commutateur (60) pour transmettre le deuxième signal électrique à au moins deux électrodes des n-m électrodes, afin de générer le champ électrique entre les électrodes avec le premier signal électrique et les électrodes avec le deuxième signal électrique. comprend :
la commande d'une première de premières unités de commutation (501) pour transmettre le premier signal électrique à une première electrode de n électrodes (10), et la commande d'une deuxième des premières unités de commutation (501) à une nième des premières unités de commutation (501) pour être désactivées ; simultanément la commande d'une première de deuxièmes unités de commutation (601) qui est connectée électriquement à la premier electrode pour être désactivée, et la commande synchrone d'au moins deux deuxièmes unités de commutation (601) parmi une deuxième des deuxièmes unités de commutation (601) à une nième des deuxièmes unités de commutation (601) pour être activées de manière séquentielle selon un ordre défini, pour transmettre le deuxième signal électrique à au moins deux électrodes de n électrodes (10) excepté à la première electrode selon l'ordre défini.

11. Procédé de commande pour le dispositif générateur de champ électrique selon la revendication 8, dans lequel m est 1, et la commande du premier ensemble commutateur (50) pour transmettre le premier signal électrique à m électrodes des n électrodes (10), et la commande du deuxième ensemble commutateur (60) pour transmettre le deuxième signal électrique à au moins deux électrodes des n-m électrodes, afin de générer le champ électrique entre les électrodes avec le premier signal électrique et les électrodes avec le deuxième signal électrique comprend :
la commande de n premières unités de commutation (501) pour être activées de manière séquentielle selon un ordre défini, afin de permettre à n électrodes (10) de recevoir le premier signal électrique de manière séquentielle selon l'ordre défini, la commande synchrone de n deuxièmes unités de commutation (601) pour être désactivées de manière séquentielle selon l'ordre défini, et la commande synchrone de combinaisons de deuxièmes unités de commutation (601) pour être activées de manière séquentielle selon l'ordre défini, pour permettre à des combinaisons d'électrodes correspondant aux combinaisons de deuxièmes unités de commutation (601) de recevoir le deuxième signal électrique de manière séquentielle selon l'ordre défini ; dans lequel chaque combinaison de deuxièmes unités de commutation comprend au moins deux deuxièmes unités de commutation (601) de n-1 deuxièmes unités de commutation (601) qui ne sont pas désactivées ; chaque combinaison d'électrodes comprend au moins deux électrodes qui ne reçoivent pas le premier signal électrique ;
dans lequel le premier ensemble commutateur (50) comprend les n premières unités de commutation (501), et le deuxième ensemble commutateur (60) comprend les n deuxièmes unités de commutation (601).

12. Procédé de commande pour le dispositif générateur de champ électrique selon la revendication 10 ou 11, dans lequel un intervalle de temps pour que les deuxièmes unités de commutation (601) soient activées ou désactivées de manière séquentielle selon l'ordre défini n'est pas inférieur à 20 millisecondes et pas supérieur à 500 millisecondes.

13. Procédé de commande pour le dispositif générateur de champ électrique selon la revendication 7, dans lequel le premier signal électrique appliqué par le générateur de signaux électriques (25) à m électrodes de n électrodes (10) est un signal haute tension, et le deuxième signal électrique appliqué par le générateur de signaux électriques (25) à au moins deux électrodes de n-m électrodes est commandé pour être un signal basse tension.

14. Support de stockage lisible par ordinateur sur lequel est stocké un logiciel, **caractérisé par le fait que** le logiciel met en œuvre, lorsqu'il est exécuté par un processeur, le procédé de commande pour le dispositif générateur de champ électrique selon l'une quelconque des revendications 7 à 13.
